# EUROPEAN PATENT SPECIFICATION

(11) **EP 4 019 973 B1**
(45) Date of publication and mention of the grant of the patent: **29.05.2024**
(21) Application number: 21217229.0
(22) Date of filing: 23.12.2021
(51) Int. Cl.: G01N 33/543, C12Q 1/6834, G01N 33/50

(54) **DETECTION METHOD OF MULTIPLE ANALYTES**
VERFAHREN ZUR DETEKTION MEHRERER ANALYTEN
MÉTHODE DE DÉTECTION DE PLUSIEURS ANALYTES

(30) Priority: 28.12.2020 US 202063130857 P; 28.05.2021 US 202163194188 P
(43) Date of publication of application: 29.06.2022
(73) Proprietor: Industrial Technology Research Institute, 310401 Hsinchu (TW)
(72) Inventor: CHIANG, Wen-Ting, 302 Zhubei City (TW); CHEN, Chien-An, 234 New Taipei City (TW); CHEN, Cheng-Tai, 320 Taoyuan City (TW)
(74) Representative: Becker, Eberhard

(56) References cited:
- EP-A1- 3 503 129
- US-A1- 2019 310 172
- DE JAGER W ET AL: "Solid-phase and bead-based cytokine immunoassay: A comparison", METHODS, ACADEMIC PRESS, NL, vol. 38, no. 4, 1 April 2006 (2006-04-01), pages 294-303, XP024908576, ISSN: 1046-2023, DOI: 10.1016/J.YMETH.2005.11.008 [retrieved on 2006-04-01]
- NINA KOLIHA ET AL: "A novel multiplex bead-based platform highlights the diversity of extracellular vesicles", JOURNAL OF EXTRACELLULAR VESICLES, vol. 5, no. 1, 1 January 2016 (2016-01-01) , page 29975, XP055485864, DOI: 10.3402/jev.v5.29975
- ALESSANDRO GORI ET AL: "Membrane-binding peptides for extracellular vesicles on-chip analysis", JOURNAL OF EXTRACELLULAR VESICLES, vol. 9, no. 1, 17 April 2020 (2020-04-17), page 1751428, XP055767519, UK ISSN: 2001-3078, DOI: 10.1080/20013078.2020.1751428
- LEE JOO HYUK ET AL: "Enhanced Cell Adhesion to the Dimpled Surfaces of Golf-Ball-Shaped Microparticles", APPLIED MATERIALS & INTERFACES, vol. 6, no. 19, 29 September 2014 (2014-09-29), pages 16493-16497, XP55923113, US ISSN: 1944-8244, DOI: 10.1021/am505997s
- HAN DI ET AL: "Engineering the Surface Pattern of Microparticles: From Raspberry-like to Golf Ball-like", APPLIED MATERIALS & INTERFACES, vol. 13, no. 26, 25 June 2021 (2021-06-25) , pages 31215-31225, XP55922932, US ISSN: 1944-8244, DOI: 10.1021/acsami.1c08663

## Description

### TECHNICAL FIELD

The disclosure relates to a detection method of multiple analytes by using a knobby microparticle.

### BACKGROUND

Ligand binding assay is a detection method that relies on affinity binding between ligand molecules and analytes, and enzyme-linked immunosorbent assay (ELISA) is the most widely used detection method. In the conventional ELISA, detection is performed by utilizing the property of binding specificity between an antibody and an antigen in combination with enzyme reaction. The technology has been developing toward maturity. However, in ELISA, long detection time is consumed and only one analyte can be detected in one reaction.

Currently on the market, products applied to multiple-protein immunoassay are based on the conventional sandwich immunoassay. Firstly, spherical microparticles with different colors are utilized to be coupled with antibodies. Next, the spherical microparticles coupled with the antibodies are performed to bind to the target antigens. Then, detection antibodies carrying specific fluorescent labels are utilized to bind to the antigens to be analyzed. Furthermore, analysis is performed with a flow cytometry analyzer. Accordingly, the purpose of detection of multiple proteins is achieved. However, in the above-mentioned multiple protein immunoassay, it is required to use microparticles of various fluorescent colors, it is required to set the fluorescence spectra for microparticles with different fluorescent colors before detection, and, during operation, it is required to distinguish particles carrying different labels before the signal analysis. On the whole, the detection time is long and the operation is complicated, which is likely to cause errors.

Therefore, it is urgent to develop a detection method in which multiple analytes can be analyzed at the same time, the operation is facilitated, and the detection sensitivity is high. DE JAGER W ET AL: "Solid-phase and bead-based cytokine immunoassay: A comparison" discloses a methods related to multiplex immunoassays for detection of cytokines and the interpretation of these.
EP 3503129 A1 discloses a magnetic particle, which includes a knobby copolymer core, a polymer layer, a magnetic substance layer and a silicon-based layer. The polymer layer covers the knobby copolymer core and has at least one functional group. In addition, the magnetic substance layer covers the polymer layer and the silicon-based layer covers the magnetic substance layer. Furthermore, a manufacturing method of the magnetic particle is also described.

### SUMMARY

The disclosure provides a detection method of multiple analytes, which can have the effect of improving detection sensitivity.

The detection method of multiple analytes in the disclosure includes the following. A microparticle is provided. According to the invention, the microparticle is a knobby microparticle. The microparticle is coupled with at least one first ligand, and includes a body including a copolymer core, a polymer layer, and a silicon-based layer from the inside to the outside, a plurality of second protrusions are formed on a surface of the copolymer core, and a plurality of first protrusions formed on a surface of the body. Next, the microparticle is mixed with a specimen including a plurality of types of analytes to form a first complex. Thereafter, the first complex is mixed with a plurality of types of second ligands carrying a plurality of types of first labels, such that the plurality of types of second ligands bind to the plurality of types of analytes in the first complex and form a second complex. Lastly, the plurality of types of first labels in the second complex are detected. A ratio of an average volume of the first protrusions to an average volume of the body is 1×10⁻⁷ to 2×10⁻², and a total volume of the first protrusions to an overall volume of the microparticle is 1×10⁻¹ to 6×10⁻¹.

In one of exemplary embodiments of the disclosure, an average height of the second protrusions is 100 nanometers to 5000 nanometers.

In one of exemplary embodiments of the disclosure, the body of the knobby magnetic particle includes a magnetic substance layer between the polymer layer and the silicon-based layer.

In one of exemplary embodiments of the disclosure, a ratio of an average height of the first protrusions to an average diameter of the body is 0.005 to 0.25.

In one of exemplary embodiments of the disclosure, an average number of the first protrusions is 5 to 500.

In one of exemplary embodiments of the disclosure, an average diameter of the microparticle is 1 µm to 20 µm.

In one of exemplary embodiments of the disclosure, the microparticle is non-spherical.

In one of exemplary embodiments of the disclosure, the manner in which the at least one first ligand is coupled to the microparticle comprises non-covalent bonding, covalent bonding, avidin-biotin interaction, electrostatic adsorption, hydrophobic adsorption, or a combination of the above.

In one of exemplary embodiments of the disclosure, the plurality of types of analytes are located on a surface of the specimen. The step of forming the first complex includes performing the at least one first ligand to recognize and directly bind to a target located on the surface of the specimen.

In one of exemplary embodiments of the disclosure, the at least one first ligand includes a first specific antibody. The first specific antibody includes an antibody against a surface antigen on the human exosome, an antibody against a surface antigen on the human blood cell, an antibody against a surface antigen on the human immune cell, an antibody against a surface antigen on the human tumor cell, or a combination thereof.

In one of exemplary embodiments of the disclosure, the specimen includes a human exosome, a human blood cell, a human immune cell, a human tumor cell, or a combination thereof. The plurality of types of analytes include a surface antigen on the human exosome, a surface antigen on the human blood cell, a surface antigen on the human immune cell, a surface antigen on the human tumor cell, or a combination thereof.

In one of exemplary embodiments of the disclosure, the plurality of types of second ligands include a plurality of types of second specific antibodies. The plurality of types of second specific antibodies include antibody against a surface antigen on the human exosome, an antibody against a surface antigen on the human blood cell, an antibody against a surface antigen on the human immune cell, an antibody against a surface antigen on the human tumor cell, or a combination thereof.

In one of exemplary embodiments of the disclosure, the at least one first ligand includes a plurality of types of first ligands. The step of forming the first complex includes performing the plurality of types of first ligands to recognize and directly bind to the plurality of types of analytes.

In one of exemplary embodiments of the disclosure, the plurality of types of first ligands include a plurality of types of nucleic acid probes. The plurality of types of nucleic acid probes include a plurality of types of primers or aptamers.

In one of exemplary embodiments of the disclosure, the plurality of types of analytes include a plurality of types of nucleic acid sequences carrying a plurality of types of second labels.

In one of exemplary embodiments of the disclosure, the plurality of types of second labels include biotin, a plurality of types of antigenic epitopes, or a combination thereof.

In one of exemplary embodiments of the disclosure, the plurality of types of second ligands include a plurality of types of specific proteins. The plurality of types of specific proteins include an anti-biotin antibody, avidin, streptavidin, neutravidin, a third specific antibody, or a combination thereof.

In one of exemplary embodiments of the disclosure, the plurality of types of first labels include a plurality of types of fluorescent labels or a plurality of types of luminescent labels.

In one of exemplary embodiments of the disclosure, the plurality of types of fluorescent labels include FITC, Alexa, PE, PerCP, BV, APC, Pacific Blue, or a combination thereof. The plurality of types of luminescent labels include luciferase.

The detection method of multiple analytes in the disclosure includes the following. A microparticle is provided. According to the invention, the microparticle is a knobby microparticle. The microparticle is coupled with a plurality of types of ligands, and includes a body including a copolymer core, a polymer layer, and a silicon-based layer from the inside to the outside, a plurality of protrusions formed on a surface of the body, and a plurality of second protrusions are formed on a surface of the copolymer core. Next, the microparticle is mixed with a specimen including a plurality of types of analytes to form a complex. The plurality of types of analytes carry a plurality of types of labels. Lastly, the plurality of types of labels in the complex are detected. A ratio of an average volume of the first protrusions to an average volume of the body is 1×10⁻⁷ to 2×10⁻², and a total volume of the first protrusions to an overall volume of the microparticle is 1×10⁻¹ to 6×10⁻¹.

In one of exemplary embodiments of the disclosure, the plurality of types of ligands include a plurality of types of nucleic acid probes. The plurality of types of labels include a plurality of types of fluorescent labels, a plurality of types of luminescent labels, or a combination thereof. The plurality of types of analytes include a plurality of types of nucleic acid sequences.

Based on the above, the microparticles of the disclosure can be arranged on the surface of the body by disposing a plurality of first protrusions with irregular shapes, thereby increasing the surface area of the microparticles (ie, the sum of the surface area of the body and the surface area of the first protrusions). In this way, the microparticles can be coupled with more first ligands to identify and bind more targets, thereby enhancing the detection signal and improving the detection sensitivity.

Several exemplary embodiments accompanied with figures are described in detail below to further describe the disclosure in details.

### BRIEF DESCRIPTION OF THE DRAWINGS

FIG. 1 is a flowchart of a detection method of multiple analytes according to an exemplary embodiment.
FIG. 2 is a schematic view illustrating flows of the detection method of multiple analytes according to an exemplary embodiment.
FIG. 3A is a schematic cross-sectional view of a knobby particle according to an exemplary embodiment.
FIG. 3B is a schematic cross-sectional view of a knobby magnetic particle according to an exemplary embodiment.
FIG. 4 is a schematic view illustrating flows of a detection method of multiple analytes according to another exemplary embodiment.
FIG. 5A to FIG. 5F are respectively graphs of specifications of spherical microparticles, which are not part of the present invention, and knobby particles analyzed by using a scanning electron microscope and a multisizer.
FIG. 6A and FIG. 6F are respectively graphs of CD9 expressions of exosomes of SKBr3 detected by using Comparative Example 1~3 and Example 1 and 3~4 coupled with anti-human CD63.
FIG. 6G and FIG. 6L are respectively graphs of CD9 expressions of exosomes of SKBr3 detected by using Comparative Example 1~3 and Example 1 and 3~4 coupled with anti-human CD9.
FIG. 7A to FIG. 7C are respectively graphs of CD9 expressions of exosomes of SKBr3 in different solutions detected by using Example 1 and Example 2 coupled with anti-human CD9.
FIG. 8A to FIG. 8C are respectively graphs of CD81 expressions of exosomes of SKBr3 in different solutions detected by using Example 1 and Example 2 coupled with anti-human CD9.
FIG. 9A to FIG. 9D are graphs of any three of CD9, CD29, CD63, and CD81 expressions of exosomes of SKBr3 detected at the same time by using Example 2 coupled with anti-human CD9.
FIG. 10A to FIG. 10D are graphs of any three of CD9, CD29, CD63, and CD81 expressions of exosomes of SKBr3 detected at the same time by using Example 2 coupled with anti-human HER2.
FIG. 11A to FIG. 11D are respectively graphs of CD9 and CD63 expressions of exosomes in clinical specimens detected at the same time by using Example 2 coupled with different first specific antibodies.
FIG. 12A to FIG. 12B are respectively graphs of AB nucleic acid to be analyzed labeled with biotin or AIV nucleic acid to be analyzed labeled with biotin detected by using a knobby magnetic particle coupled with an AIV probe.

### DETAILED DESCRIPTION

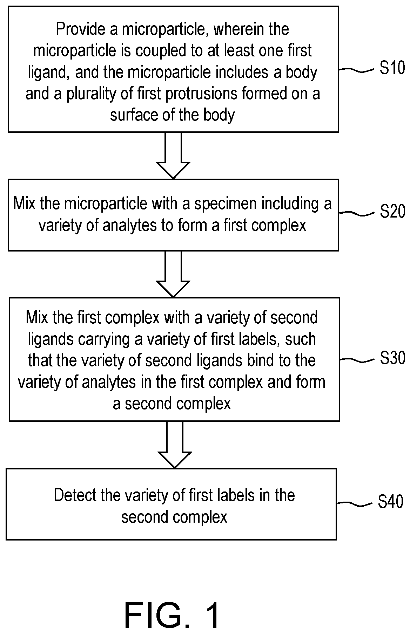

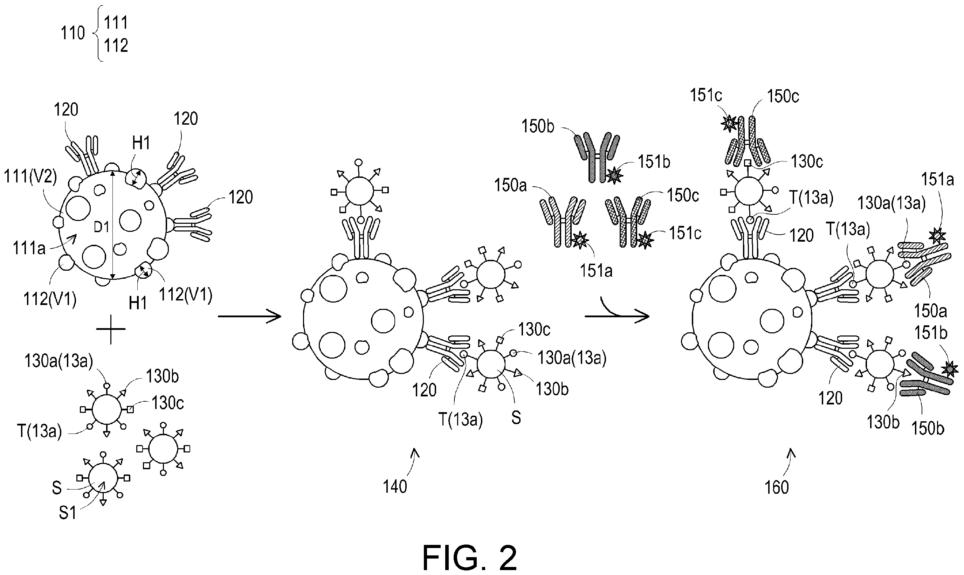

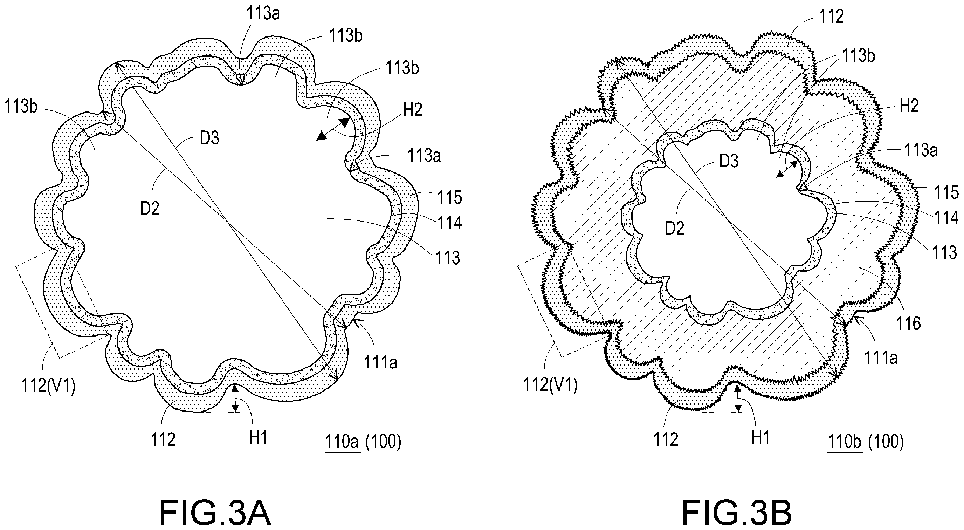

FIG. 1 is a flowchart of a detection method of multiple analytes according to an exemplary embodiment. FIG. 2 is a schematic view illustrating flows of the detection method of multiple analytes according to an exemplary embodiment. FIG. 3A is a schematic cross-sectional view of a knobby particle according to an exemplary embodiment. FIG. 3B is a schematic cross-sectional view of a knobby magnetic particle according to an exemplary embodiment.

With reference to FIG. 1, FIG. 2, FIG. 3A, and FIG. 3B together, step S10 is firstly performed, in which a microparticle 110 is provided. According to the invention, the microparticle 110 is a knobby microparticle. The microparticle 110 includes a body 111 and a plurality of first protrusions 112 formed on a surface 111a of the body 111. Accordingly, the microparticle 100 is non-spherical. In terms of appearance, the first protrusions 112 have irregular shapes, and may be evenly or unevenly distributed on the surface 111a of the body 111. Substantially, the first protrusions 112 are integrally formed with the body 111, and the first protrusions 112 are seamlessly connected to the body 111. In this embodiment, the first protrusions 112 have an average height H1 (i.e., the average vertical distance from the top of the first protrusions 112 to the surface 111a of the body 111).

Specifically, with reference to FIG. 2, FIG. 3A, and FIG. 3B together, in this embodiment, the knobby microparticle 110 may be a knobby particle 110a or a knobby magnetic particle 110b. The knobby particle 110a is non-magnetic, and the knobby magnetic particle 110b is magnetic. As shown in FIG. 3A, the body 111 of the knobby particle 110a includes a copolymer core 113, a polymer layer 114, and a silicon-based layer 115 from the inside to the outside. A plurality of second protrusions 113b are formed on a surface 113a of the copolymer core 113. The second protrusions 113b have irregular shapes, and may be evenly or unevenly distributed on the surface 113a of the copolymer core 113. Substantially, the second protrusions 113b are integrally formed with the copolymer core 113, and the second protrusions 113b are seamlessly connected to the copolymer core 113. In this embodiment, an average height H2 of the second protrusions 113b (i.e., the average vertical distance from the top of the second protrusions 113b to the surface 113a of the copolymer core 113) may be 100 nanometers (nm) to 5000 nm, for example but not limited to, about 200 nm to 4500 nm, about 500 nm to 4000 nm, about 800 nm to 3500 nm, about 300 nm to 1000 nm, about 600 nm to 1800 nm, about 750 nm to 2500 nm, about 900 nm to 3000 nm, about 1000 nm to 3600 nm, and about 1500 nm to 4800 nm. In this embodiment, the polymer layer 114 and the silicon-based layer 115 are sequentially formed on the copolymer core 113 in a substantially conformal manner. Therefore, the first protrusions 112 of the knobby particle 110a substantially correspond to the second protrusions 113b of the copolymer core 113, but not limited thereto.

As shown in FIG. 3B, the body 111 of the knobby magnetic particle 110b includes the copolymer core 113, the polymer layer 114, a magnetic substance layer 116, and the silicon-based layer 115 from the inside to the outside. The second protrusions 113b is formed on the surface 113a of the copolymer core 113. The second protrusions 113b have irregular shapes, and may be evenly or unevenly distributed on the surface 113a of the copolymer core 113. Substantially, the second protrusions 113b are integrally formed with the copolymer core 113, and the second protrusions 113b are seamlessly connected to the copolymer core 113. In this embodiment, the average height H2 of the second protrusions may be 100 nm to 5000 nm, for example but not limited to, about 200 nm to 4500 nm, about 500 nm to 4000 nm, about 800 nm to 3500 nm, about 300 nm to 1000 nm, about 600 nm to 1800 nm, about 750 nm to 2500 nm, about 900 nm to 3000 nm, about 1000 nm to 3600 nm, and about 1500 nm to 4800 nm. In this embodiment, the polymer layer 114, the magnetic substance layer 116, and the silicon-based layer 115 are sequentially formed on the copolymer core 113 in a substantially conformal manner. The first protrusions 112 of the knobby magnetic particle 110b substantially corresponds to the second protrusions 113b of the copolymer core 113, but not limited thereto.

In this embodiment, the copolymer core 113 is, for example but not limited to, styrene/divinylbenzene copolymer, methyl methacrylate/triethylene glycol dimethacrylate copolymer, methyl methacrylate/ethylene glycol dimethacrylate copolymer, styrene/triethylene glycol dimethacrylate copolymer, styrene/ethylene glycol dimethacrylate copolymer, or methyl methacrylate/divinylbenzene copolymer. The polymer layer 114 may be a surface of the copolymer core 113 modified with functional groups, and includes at least one functional group (for example but not limited to, a carboxyl group, an amino group, or a combination thereof). The material of the silicon-based layer 115 may include but is not limited to tetramethoxysilane (TMOS), tetraethoxysilane (TEOS), 3-Aminopropyltriethoxysilane (APTES), and 3-Glycidoxypropyltrimethoxysilane (GOPTS). The material of the magnetic substance layer 116 may include but is not limited to paramagnetic materials, superparamagnetic materials, ferromagnetic materials, ferrimagnetic materials, or a combination thereof.

With further reference to FIG. 2, FIG. 3A, and FIG. 3B, in this embodiment, the average height H1 of the first protrusions 112 may be 0.1 micrometer (µm) to 5 µm, for example but not limited to, about 0.2 µm to 4.5 µm, about 0.3 µm to 4 µm, about 0.4 µm to 3.5 µm, about 0.5 µm to 3 µm, and about 0.6 µm to 2.5 µm. An average volume V1 of the first protrusions 112 may be 0.00052 cubic micrometer (µm³) to 65 µm³, for example but not limited to, about 0.001 µm³ to 60 µm³, about 0.01 µm³ to 50 µm³, and about 0.1 µm³ to 40 µm³. An average diameter D1 of the body 111 may be 1 µm to 20 µm, for example but not limited to, about 2 µm to 15 µm, about 3 µm to 18 µm, about 4 µm to 10 µm, and about 5 µm to 12 µm. An average volume V2 of the body 111 may be 0.52 µm³ to 4200 µm³, for example but not limited to, about 1 µm³ to 4000 µm³, about 10 µm³ to 3800 µm³, about 20 µm³ to 3600 µm³, about 30 µm³ to 3300 µm³, about 40 µm³ to 2900 µm³, about 50 µm³ to 2500 µm³, about 60 µm³ to 2300 µm³, about 80 µm³ to 2000 µm³, and about 100 µm³ to 1000 µm³. A ratio of the average height H1 of the first protrusions 112 to the average diameter D1 of the body 111 may be 0.005 to 0.25, for example but not limited to, about 0.001 to 0.23, about 0.05 to 0.20, about 0.01 to 0.18, and about 0.05 to 0.15. According to the invention, a ratio of the average volume V1 of the first protrusions 112 to the average volume V2 of the body 111 is 1×10⁻⁷ to 2×10⁻², for example but not limited to, 1×10⁻⁶ to 1.5×10⁻², 1×10⁻⁵ to 1.2×10⁻², and 1×10⁻⁵ to 1×10⁻². The ratio of the total volume of the first protrusions to an overall volume of the knobby microparticle is 1×10⁻¹ to 6×10⁻¹. An average diameter D of the microparticle 110 (i.e., the average of the shortest diameter and the longest diameter of the microparticle 110, for example, the average of a shortest diameter D2 and a longest diameter D3 of the knobby particle 110a or of the knobby magnetic particle 110b) may be 1 µm to 20 µm, for example but not limited to, about 2 µm to 18 µm, about 3 µm to 15 µm, and about 5 µm to 10 µm.

With further reference to FIG. 2, in this embodiment, the microparticle 110 is coupled with at least one first ligand 120 (one first ligand shown in FIG. 1 serving as an example, but not limited thereto). The first ligand 120 is coupled to the surface 111a of the body 111 of the microparticle 110 and the first protrusions 112. The manner in which the first ligand 120 is coupled to the microparticle 110 includes non-covalent bonding, covalent bonding, avidin-biotin interaction, electrostatic adsorption, hydrophobic adsorption, or a combination thereof. In this embodiment, the first ligand 120 includes, for example but not limited to, a specific antibody.

Next, with further reference to FIG. 1 and FIG. 2, step S20 is performed, in which the microparticle 110 is mixed with a plurality of types of analytes 130a, 130b, 130c to form a first complex 140. In this embodiment, the plurality of types of analytes 130a, 130b, 130c are located on a surface S1 of a specimen S. Forming the first complex 140 includes performing the at least one first ligand 120 to recognize and directly bind to a target T on the surface S1 of the specimen S.

In this embodiment, the first ligand 120 includes a first specific antibody, which is capable of recognizing and directly binding to the target T. The first specific antibody includes but is not limited to an antibody against a surface antigen on the human exosome (e.g., anti-human CD9, anti-human CD63, anti-human CD29, anti-human CD81, anti-human HER2, anti-human EGFR, and anti-human EpCAM), an antibody against a surface antigen on the human blood cell (e.g., anti-human CD45 and anti-human CD235a), an antibody against a surface antigen on the human immune cell (e.g., anti-human CD3 and anti-human CD19), an antibody against a surface antigen on the human tumor cell (e.g., anti-human CEA, anti-human PD-L1, and anti-human HER2) or a combination thereof.

In this embodiment, the specimen S includes but is not limited to human exosomes, human blood cells, human immune cells, human tumor cells, or a combination thereof. The analytes 130a, 130b, 130c include but is not limited to a surface antigen on the human exosomes (e.g., CD9, CD63, CD81, HER2, EGFR, and EpCAM), a surface antigen on the human blood cells (e.g., CD45 and CD235a), a surface antigen on the human immune cells (e.g., CD3 and CD19), a surface antigen on the human tumor cells (e.g., CEA, PD-L1, and HER2), or a combination thereof.

In this embodiment, the target T may include but is not limited to at least one of the surface antigens on the human exosome, at least one of the surface antigens on the human blood cell, at least one of the surface antigens on the human immune cell, at least one of the surface antigens on the human tumor cell, or a combination thereof. The target T may be identical or different to any one of the analytes 130a, 130b, 130c. For example, in an embodiment, the target T is, for example, the surface antigen CD9 on the human exosomes, and the analytes are, for example, the surface antigens CD9, CD63, and CD81 on the human exosomes. In another embodiment, the target T is, for example, the surface antigen HER2 on the human exosomes, and the analytes are, for example, the surface antigens CD9, CD63, and CD81 on the human exosomes. Nonetheless, the disclosure is not limited thereto. Notably, even if the target T and the analyte are the same kind of surface antigens, the target T and the analyte 130a are not the same surface antigen. For example, as shown in FIG. 2, the target T and the analyte 130a are the same kind of surface antigen 13a, in which the part that is recognized and bound to by the first ligand 120 is namely the target T, while another part that is not bound to by the first ligand 120 may serve as the analyte 130a for subsequent detection.

Then, step S30 is performed, in which the first complex 140 is mixed with a plurality of types of second ligands 150a, 150b, 150c carrying a plurality of types of first labels 151a, 151b, 151c, such that the second ligands 150a, 150b, 150c bind to the analytes 130a, 130b, 130c in the first complex 140 and form a second complex 160. For the sake of clarity, only one specimen S binding to one second ligand (150a, 150b, or 150c) is shown in FIG. 2. In fact, the specimen S may bind to the plurality of types of (or the plurality of) second ligands 150a, 150b, 150c at the same time as long as the plurality of types of (or the plurality of) analytes 130a, 130b, 130c are present on the surface of the specimen S.

In this embodiment, the second ligands 150a, 150b, 150c are, for example, a plurality of types of second specific antibodies. In this embodiment, the second specific antibodies include an antibody against a surface antigen on the human exosome, an antibody against a surface antigen on the human blood cell, an antibody against a surface antigen on the human immune cell, an antibody against a surface antigen on the human tumor cell, or a combination thereof. The second specific antibodies may be identical or different to the first specific antibodies. The first labels 151a, 151b, 151c include but are not limited to a plurality of types of different fluorescent labels or luminescent labels. The fluorescent labels may include but are not limited to FITC, Alexa (e.g., AF-488, AF-594, AF-647, and AF-700), PE (e.g., PE, PE-Cyanine5, PE-Cyanine7, and PE-Dazzle594), PerCP (e.g., PerCP and PerCP-Cyanine5.5), BV (Brilliant Violet, e.g., BV421, BV450, BV510, BV570, BV605, BV650, BV711, BV750, and BV785), APC (e.g., APC and APC-Cyanine7), Pacific Blue, or a combination thereof. The luminescent labels include but are not limited to luciferase. Those having common knowledge in the technical field may select the first ligand, the first label, and the second ligand depending on actual needs (e.g., the source of specimens or the number of analytes), which is not limited by the disclosure.

Lastly, step S40 is performed, in which the first labels 151a, 151b, 151c of the second ligands 150a, 150b, 150c in the second complex 160 are detected. The detection result may represent the relative contents (or expressions) of the analytes 130a, 130b, 130c on the surface S1 of the specimen S. In this embodiment, the detection is performed by using, for example, a flow cytometry analyzer, but is not limited thereto. So far, the performing of the detection method of multiple analytes of this embodiment has been substantially completed.

In this embodiment, compared to a general spherical microparticle, in the knobby microparticle 110 of this embodiment, by disposing the first protrusions having irregular shapes on the surface of the body, the surface area of the microparticle 110 (i.e., the sum of the surface area of the body and the surface area of the first protrusions) is thus increased. Accordingly, the microparticle 110 of this embodiment may be coupled with more first ligands 120 to recognize and bind to more targets T, thereby increasing the strength of detection signals to improve the detection sensitivity.

Besides, in this embodiment, since the surface of the magnetic substance layer 140 of the knobby magnetic particle 110b is a rough surface (or has small protrusions), the surface of the knobby magnetic particle 110b (i.e., the surface 111a of the body 111 and the surface of the first protrusions 112) is also a rough surface. Next, compared to the knobby particle 110a, since the surface of the knobby magnetic particle 110b is a rough surface, the knobby magnetic particle 110b is of a greater surface area. Accordingly, the knobby magnetic particle 110b is coupled with more first ligands 120, and recognizes and binds to more targets T. In addition, more analytes 130a, 130b, 130c are detected, and the strength of detection signals can be increased and the detection sensitivity can be improved. Moreover, since the knobby magnetic particle 110b is magnetic, the detection time can be reduced, and the efficiency and convenience of detection can be improved.

Hereinafter, other embodiments will be described. Note that, the reference numerals and part of the contents of the foregoing embodiments will remain to be used in the following embodiments, where the same reference numerals are used to denote the same or similar elements, and the description of the same technical contents is omitted. Reference may be made to the foregoing embodiments for the description of the omitted part, which will not be repeated in the following embodiments.

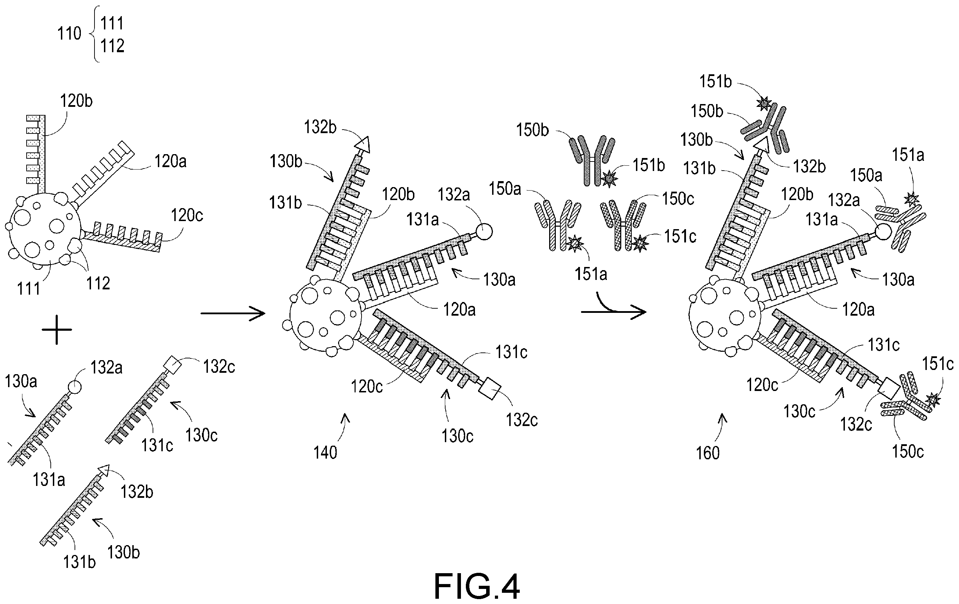

FIG. 4 is a schematic view illustrating flows of a detection method of multiple analytes according to another embodiment of the disclosure. With reference to FIG. 2 and FIG. 4 together, the method of this embodiment is similar to the method in FIG. 2, while the main difference lies in that, the at least one first ligand 120 includes a plurality of types of first ligands 120a, 120b, 120c, and the first ligands 120a, 120b, 120c are, for example, a plurality of types of nucleic acid probes (e.g., a plurality of types of primers or aptamers). The analytes 130a, 130b, 130c include a plurality of types of nucleic acid sequences carrying a plurality of types of second labels 132a, 132b, 132c (for example but not limited to biotin, antigenic epitopes, or a combination thereof). The second ligands 150a, 150b, 150c include specific proteins (for example but not limited to, an anti-biotin antibody, avidin, streptavidin, neutravidin, third specific antibody, or a combination thereof).

Specifically, with reference to FIG. 4, the microparticle 110 coupled with the first ligand 120a, 120b, 120c is first mixed with the analytes 130a, 130b, 130c, such that the first ligands 120a, 120b, 120c recognize and directly bind to the corresponding (or complementary) parts of sequence 131a, 131b, 131c of the analytes 130a, 130b, 130c and form the first complex 140. Next, the first complex 140 is mixed with the second ligands 150a, 150b, 150c carrying the first labels 151a, 151b, 151c, such that the second ligands 150a, 150b, 150c bind to the second labels 132a, 132b, 132c of the analytes 130a, 130b, 130c in the first complex 140 and form the second complex 160. Lastly, the first labels 151a, 151b, 151c of the second ligands 150a, 150b, 150c in the second complex 160 are detected.

Besides, in some other embodiments, the second labels 132a, 132b, 132c may also be the first labels 150a, 150b, 150c. Accordingly, after the first ligands 120a, 120b, 120c bind to the parts of sequence 131a, 131b, and 131c of the analytes 130a, 130b, 130c, the formed first complex 140 carries a plurality of types of fluorescent labels or a plurality of types of luminescent labels. Therefore, the detection may be performed directly without adding the second ligand to form the second complex. So far, the performing of the detection method of multiple analytes of this embodiment has been substantially completed.

Hereinafter, some embodiments of the disclosure accompanied with the drawings will be described. However, the following embodiments and accompanying drawings only serve for aiding the description, instead of limiting the disclosure.

### [EMBODIMENTS]

### Embodiment 1: specification analysis of knobby particles

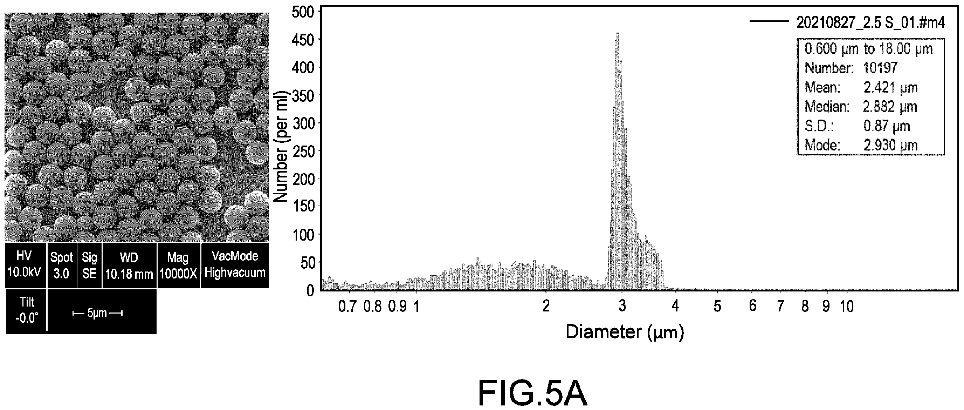

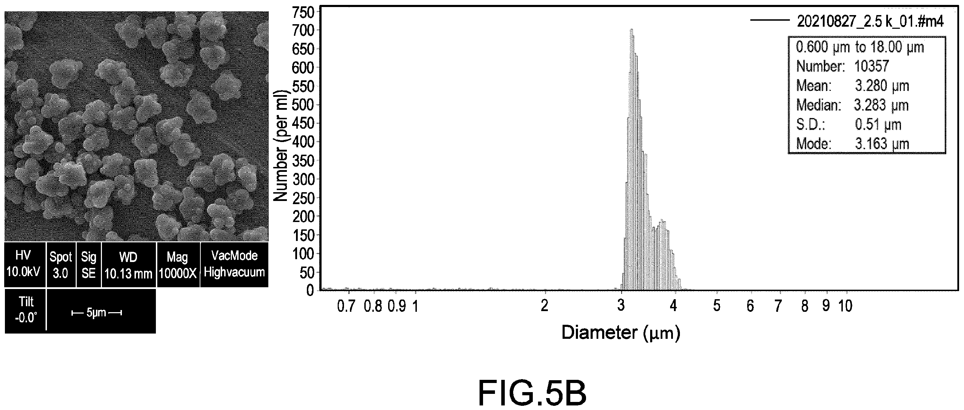

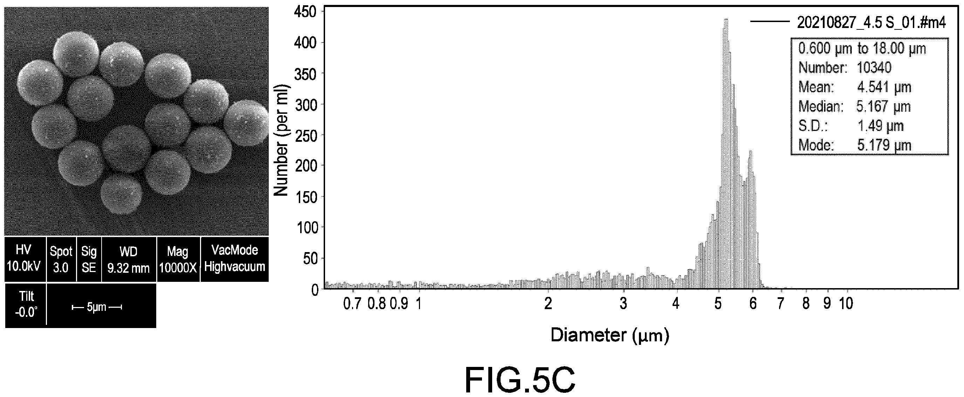

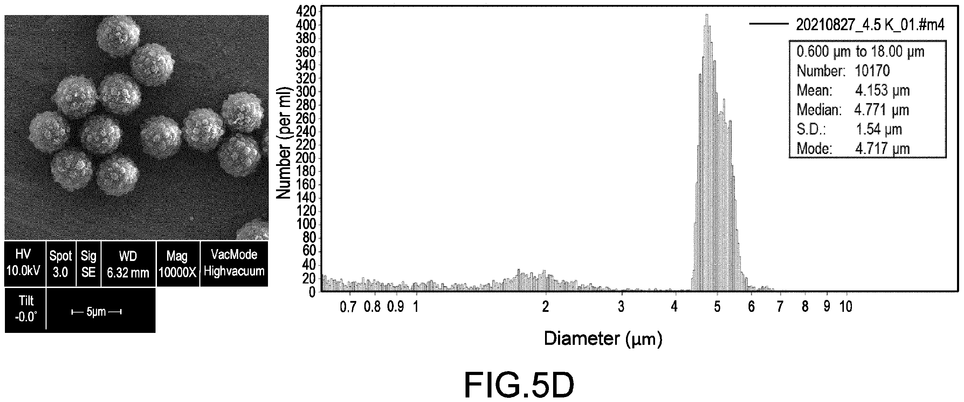

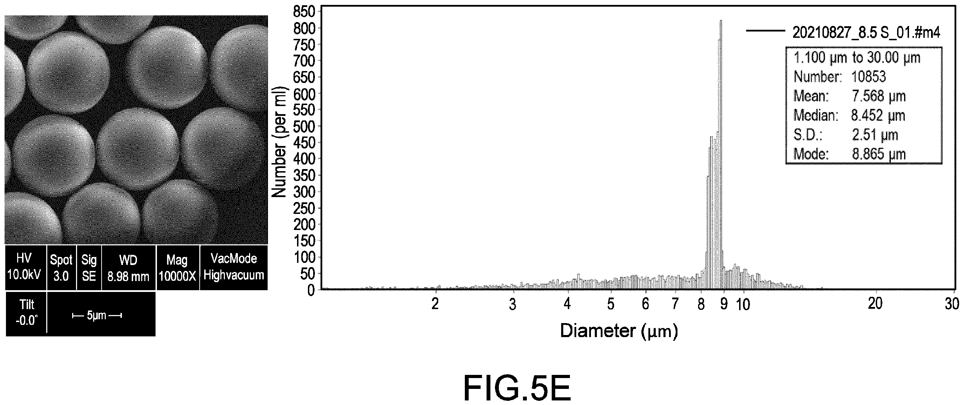

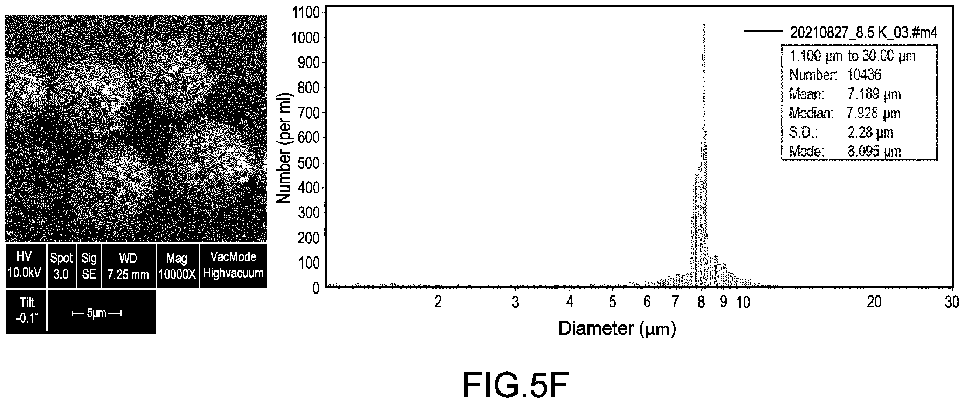

In this embodiment, specifications of general spherical microparticles or knobby particles are analyzed by using a scanning electron microscope (SEM) and a multisizer. The analysis results are shown in FIG. 5A to FIG. 5F, in which FIG. 5A, FIG. 5C and FIG. 5E are the analysis results of the spherical microparticles, which are not part of the present invention, and FIG. 5B, FIG. 5D and FIG. 5F are the analysis results of the knobby particles.

According to the analysis results of FIG. 5A to FIG. 5F, the average diameter of the spherical microparticles in FIG. 5A is about 2.421 µm, the average diameter of the knobby particles in FIG. 5B is about 3.280 µm, the average diameter of the spherical microparticles in FIG. 5C is about 4.541 µm, the average diameter of the knobby particles in FIG. 5D is about 4.153 µm, the average diameter of the spherical microparticles in FIG. 5E is about 7.568 µm, and the average diameter of the knobby particles in FIG. 5F is about 7.189 µm.

Next, further analysis of the specifications of the knobby particles of FIGs. 5B, 5D and 5F is performed. In detail, as shown in FIG. 5B, the average volume of the knobby particles is about 13.86 µm³, the average diameter of the bodies is about 2.5 µm, the average volume of the bodies is about 6.14 µm³, the average height of the first protrusions is about 1.2 µm, the average volume of the first protrusions is about 0.679 µm³, and the total volume of the first protrusions is about 7.72 µm³. That is, in the knobby particles of FIG. 5B, the ratio of the average height of the first protrusions to the average diameter of the bodies is about 0.48, the ratio of the average volume of the first protrusions to the average volume of the bodies is about 0.111, each knobby particle has about 11 first protrusions, and the total volume of the first protrusions is 55.7% of the average volume of the knobby particles.

In FIG. 5D, the average volume of the knobby particles is about 49.09 µm³, the average diameter of the bodies is about 4.5 µm, the average volume of the bodies is about 35.78 µm³, the average height of the first protrusions is about 0.8 µm, the average volume of the first protrusions is about 0.268 µm³, and the total volume of the first protrusions is about 13.31 µm³. That is, in the knobby particles of FIG. 5D, the ratio of the average height of the first protrusions to the average diameter of the bodies is about 0.18, the ratio of the average volume of the first protrusions to the average volume of the bodies is about 0.007, each knobby particle has about 49 first protrusions, and the total volume of the first protrusions is 27.1% of the average volume of the knobby particles.

In FIG. 5F, the average volume of the knobby particles is about 201 µm³, the average diameter of the bodies is about 7.5 µm, the average volume of the bodies is about 166 µm³, the average height of the first protrusions is about 1 µm, the average volume of the first protrusions is about 0.392 µm³, and the total volume of the first protrusions is about 35 µm³. That is, in the knobby particles of FIG. 5F, the ratio of the average height of the first protrusions to the average diameter of the bodies is about 0.133, the ratio of the average volume of the first protrusions to the average volume of the bodies is about 0.0024, each knobby particle has about 89 first protrusions, and the total volume of the first protrusions is 17.4% of the average volume of the knobby particles.

In particular, in the knobby particles, the ratio of the average height (or average volume) of the first protrusions to the average diameter (or average volume) of the bodies may be related to the surface morphology (ie, overall appearance). For example, in the knobby particles of FIG. 5B, the ratio of the average height of the first protrusions to the average diameter of the body is greater than 0.25, and the ratio of the average volume of the first protrusions to the average volume of the bodies is greater than 2×10⁻². Therefore, the surface morphology of the knobby particles in FIG. 5B are irregular. In other words, it is difficult to clearly identify the positions of the bodies and the first protrusions. On the contrary, in the knobby particles of FIG. 5D and FIG. 5F, the ratios of the average height of the first protrusions to the average diameter of the bodies are between 0.005 and 0.25, and the ratios of the average volume of the first protrusions to the average volume of the bodies are between 1×10⁻⁷ and 2×10⁻². Therefore, the positions of the bodies and the first protrusions may be identified in the knobby particles of FIG. 5D and FIG. 5F.

### Embodiment 2: assay for the number of grafted specific antibodies

In this embodiment, the first specific antibody (anti-human CD9, anti-human CD63, or anti-human HER2) was firstly coupled to a general spherical microparticle, which is not part of the present invention, and a microparticle (a knobby particle or a knobby magnetic particle) of this disclosure. Next, assays and comparisons of the number of grafted specific antibodies were performed on the spherical microparticle coupled with the first specific antibody, the knobby particle coupled with the first specific antibody, and the knobby magnetic particle coupled with the first specific antibody. The classes and diameters of the microparticles used in this embodiment are shown in Table 1.

**Table 1: the classes and diameters of the microparticles**

| | Class of the microparticle | Diameter of the microparticles (µm) |
|---|---|---|
| Example 1 | knobby particle | 8.5 |
| Example 2 | knobby magnetic particle | 8.5 |
| Example 3 | knobby particle | 2.5 |
| Example 4 | knobby particle | 4.5 |
| Comparative Example 1 | spherical microparticle | 8.5 |
| Comparative Example 2 | spherical microparticle | 2.5 |
| Comparative Example 3 | spherical microparticle | 4.5 |

In this embodiment, the step in which anti-human CD9 (or anti-human CD63 or anti-human HER2) was coupled to the spherical microparticle, the knobby particle, and the knobby magnetic particle is generally as follows: (1) 1×10⁶ particles (i.e., the spherical microparticles of Comparative Examples 1 to 3, the knobby particles of Examples 1 and 3 to 4, or the knobby magnetic particles of Examples 2) surface-modified with amine groups were washed with 200 µL of a MES buffer solution three times. Next, 20 mg of EDC (1-ethyl-3-(3-dimethylaminopropyl) carbodiimide hydrochloride), 20 mg of NHS (N-Hydroxysulfosuccinimide sodium salt), and 4 mg of PAA (15 kDa poly acrylic acid) were dissolved in 400 µL of a MES buffer solution, and were mixed with the washed particles. Next, at room temperature, the mixing was performed with a vortex mixer at a rotation speed of 1000 rpm for reaction for 30 minutes. Next, the particles were collected. The spherical microparticles and the knobby particles were collected by centrifugation at a rotation speed of 10000 rpm for 3 minutes, and the knobby magnetic particles were collected with a magnet, in which the magnet was performed to stay thereon for at least 1 minute. After the reaction solution was removed, the particles were washed with 200 µL of a MES buffer solution three times and added with 20 µL of anti-human CD9 (or anti-human CD63 or anti-human HER2) into a pH3 citric acid-PBS solution (0.625M citric acid dissolved in PBS solution, pH3.0) for reaction overnight at 4°C, such that anti-human CD9 (or anti-human CD63 or anti-human HER2) was coupled on the surface of the particles. Then, the mixture was added into 200 µL of a bovine serum albumin solution (10 mg/mL BSA dissolved in a MES solution) for perform reaction overnight at 4°C to cover the rest of the surface of the particles that has not been coupled with anti-human CD9 (or anti-human CD63 or anti-human HER2). (2) After the reaction was completed, the particles were collected. The spherical microparticles and the knobby particles were collected by centrifugation at a rotation speed of 10000 rpm for 3 minutes. The knobby magnetic particles were collected by a magnet and the magnet was performed to stay thereon for at least 1 minute each time. The reaction solution was removed. The particles were washed with a PBS solution (0.1% BSA and 0.01% sodium azide dissolved in PBS) three times. Lastly, the particles coupled with anti-human CD9 (or anti-human CD63 or anti-human HER2) were dispersed in 100 µL of a PBS solution. (3) The concentration of particles was calculated by using an automated cell counter, and particles coupled with anti-human CD9 (or anti-human CD63 or anti-human HER2) on the surface with a concentration of about 3 to 8×10⁶/mL were obtained.

Next, the step in which assays of the number of grafted specific antibodies were performed on the particles coupled with anti-human CD9 (or anti-human CD63 or anti-human HER2) is generally as follows: (1) 50 µL (1250 counts) of the particles coupled with anti-human CD9 (or anti-human CD63 or anti-human HER2) on the surface were added into 5 µL of Antimouse IgG-FITC, and then were added into a PBS solution until the total reaction volume was 200 µL. At room temperature, the mixing was performed with a vortex mixer at a rotation speed of 1500 rpm for reaction for 30 minutes. After the reaction was completed, the particles were collected. The spherical microparticles and knobby particles were collected by centrifugation at a rotation speed of 10000 rpm for 3 minutes. The knobby magnetic particles were collected with a magnet and stayed on the magnet for at least 1 minute. (2) The reaction solution was removed, and the collected particles were washed with 200 µL of a PBS solution two times. (3) After washing, the particles were added into 100 µL of a PBS solution, and fluorescence signal analysis was performed on the particles with a flow cytometry analyzer to measure the number of grafted anti-human CD9 (or anti-human CD63 or anti-human HER2) (represented by average fluorescence intensity, i.e., as the average fluorescence intensity increases, the number of grafts increases). (4) Statistical analysis was performed, and the test results were expressed in multiples of fluorescence intensity. In Table 2, comparisons of the grafting number of spherical microparticles (Comparative Example 1), knobby particles (Example 1) and knobby magnetic particles (Example 2) with the same diameter (8.5 µm) is illustrated. Table 3 shows comparisons of the grafting number of spherical microparticles (Comparative Examples 1 to 3) and knobby particles (Examples 1, 3 to 4) with different diameters (i.e., 2.5, 4.5, 8.5 µm). Table 4 shows comparisons of the grafting number of spherical microparticles (Comparative Examples 1 to 3) and knobby particles (Examples 1, 3 to 4) with the same diameter (i.e., 2.5, 4.5, 8.5 µm).

**Table 2: comparison of the number of grafted first specific antibodies (anti-human CD9, anti-human CD63 or Anti-Human Her2) in Comparative Example 1, Example 1 and Example 2**

| | Multiples of fluorescence intensity | |
|---|---|---|
| First specific antibody | Example 1/Comparative Example 1 (diameter: 8.5 µm) | Example 2/Example 1 (diameter: 8.5 µm) |
| Anti-Human CD9 | ∼1.71 | ∼2.24 |
| Anti-Human CD63 | ∼4.55 | - |
| Anti-Human Her2 | - | ∼3.81 |

According to the results in Table 2, when the first specific antibody is anti-human CD9, the number of grafts in Example 1 is about 1.71 times that in Comparative Example 1. When the first specific antibody is anti-human CD63, the number of grafts in Example 1 is about 4.55 times that in Comparative Example 1. That is, whether the first specific antibody is anti-human CD9 or anti-human CD63, the number of grafts in Example 1 is greater than the number of grafts in Comparative Example 1. Therefore, compared with a general spherical microparticle, the knobby particle of this disclosure is significantly coupled with more first specific antibodies.

In addition, when the first specific antibody is anti-human CD9, the number of grafts in Example 2 is about 2.24 times that in Example 1. When the first specific antibody is anti-human HER2, the number of grafts in Example 2 is about 3.81 times that in Example 1. That is, whether the first specific antibody is anti-human CD9 or anti-human HER2, the number of grafts in Example 2 is greater than the number of grafts in Example 1. Therefore, compared with the knobby particle of this disclosure, the knobby magnetic particle of this disclosure is significantly coupled with more first specific antibodies.

**Table 3: comparison of the number of grafted first specific antibodies (anti-human CD9 or Anti-Human CD63) in Comparative Examples 1 to 3 and Examples 1 and 3 to 4**

| | Multiples of fluorescence intensity | | | | |
|---|---|---|---|---|---|
| First specific antibody | Comparative Example 3 / Comparative Example 2 | Comparative Example 1 / Comparative Example 2 | Example 1 / Comparative Example 2 | Example 4 / Comparative Example 2 | Example 1 / Comparative Example 2 |
| Anti-Human CD9 | ∼4.71 | ∼5.62 | ∼0.90 | ∼6.05 | ∼9.59 |
| Anti-Human CD63 | ∼1.96 | ∼1.98 | ∼0.63 | ∼3.16 | ∼9.00 |

**Table 4: comparison of the number of grafted first specific antibodies (anti-human CD9 or Anti-Human CD63) in Comparative Examples 1 to 3 and Examples 1 and 3 to 4**

| | Multiples of fluorescence intensity | | |
|---|---|---|---|
| First specific antibody | Example 3 /Comparative Example 2 (diameter: 2.5 µm) | Example 4 /Comparative Example 3 (diameter: 4.5 µm) | Example 1 /Comparative Example 1 (diameter: 8.5 µm) |
| Anti-Human CD9 | ∼0.90 | ∼1.28 | ∼1.71 |
| Anti-Human CD63 | ∼0.63 | ∼1.61 | ∼4.55 |

According to the results in Table 3, whether it is spherical microparticles or knobby particles, when the particle size is larger, the surface area that can be coupled to the first specific antibody increases accordingly, so the grafting number of the first specific antibody can be increased. According to the results in Table 4, compared with spherical microparticles (Comparative Example 3 or Comparative Example 1), knobby particles with similar particle sizes (Example 4 or Example 1) can couple significantly more first-specific antibodies. Compared with the spherical microparticles (Comparative Example 2) with a diameter of about 2.5 µm, the knobby particles (Example 3) with a diameter of about 2.5 µm may be due to uneven surface morphology (please refer to Figure 5B), resulting in a lower detected fluorescent signal.

### Embodiment 3: detection of surface antigens on exosomes of breast cancer cell lines SKBr3

In this embodiment, the expression of exosome surface antigens on the surface of exosomes was detected by using a spherical microparticle (Comparative Examples 1, 2 and 3), a knobby particle (Examples 1, 3 and 4), and a knobby magnetic particle (Example 2) coupled with a first specific antibody (anti-human CD9 or anti-human CD63), and using a second specific antibody (anti-human CD9-Alexa488 or anti-human CD81-APC) carrying fluorescent labels. Comparative Example 1 is taken as an example for description. Specifically, Comparative Example 1 coupled with anti-human CD9 (or anti-human CD63) was first mixed with exosomes carrying a plurality of types of exosome surface antigens to allow anti-human CD9 (or anti-human CD63) to recognize and directly bind to CD9 (or CD63) located on the surface of the exosomes to form a first complex. Next, the first complex was mixed with anti-human CD9-Alexa488 (or anti-human CD81-APC) to allow anti-human CD9-Alexa488 (or anti-human CD81-APC) to bind to CD9 (or CD81) on the exosome surface in the first complex and form a second complex. Then, fluorescence signal analysis of the second complex was performed with a flow cytometry analyzer to detect the expression of exosome surface antigens. The exosomes were exosomes purified from breast cancer cell lines (SKBr3). The above-mentioned spherical microparticles included spherical microparticles having an average diameter of about 2.5 µm (Comparative Example 2), spherical microparticles having an average diameter of about 4.5 µm (Comparative Example 3), and spherical microparticles having an average diameter of about 8.5 µm (Comparative Example 1). The above-mentioned knobby particles included knobby particles having an average diameter of about 2.5 µm (Example 3), knobby particles having an average diameter of about 4.5 µm (Example 4), and knobby particles having an average diameter of about 8.5 µm (Example 1). The above-mentioned knobby magnetic particles included knobby magnetic particles having an average diameter of about 8.5 µm (Example 2).

In this embodiment, the step in which Comparative Examples 1 to 3, Examples 1 and 3 to 4, or Example 2 coupled with anti-human CD9 (or anti-human CD63) was mixed with the exosomes of SKBr3, such that anti-human CD9 (or anti-human CD63) recognized and directly bound to CD9 (or CD63) on the surface of the exosomes to form the first complex is generally as follows: 50 µL (1250 counts) of Comparative Examples 1 to 3, Examples 1 and 3 to 4, or Example 2 was reacted with exosomes of SKBr3 with a volume of 20 to 50 µL, then added into a PBS solution until the total reaction volume was 200 µL, and then mixed and reacted at room temperature for 90 minutes. After the reaction was completed, the first complex containing Comparative Examples 1 to 3 or the first complex of Examples 1 and 3 to 4 was collected by centrifugation at a rotation speed of 10000 rpm for 3 minutes, and the first complex containing Example 2 was collected with a magnet and stayed on the magnet for at least 1 minute. The reaction solution was removed and the first complex was repetitively washed with 200 µL of a PBS solution two times.

In this embodiment, the step in which the first complex was mixed with anti-human CD9-Alexa488 (or anti-human CD81-APC), such that anti-human CD9-Alexa488 (or anti-human CD81-APC) bound to CD9 (or CD81) on the surface of the exosomes in the first complex and formed the second complex is generally as follows: 100 µL of anti-human CD9-Alexa488 (or anti-human CD81-APC) was added into the first complex. Then, at room temperature, the mixing was performed with a vortex mixer at a rotation speed of 1500 rpm for reaction for 30 minutes to form the second complex. After the reaction was completed, the second complex containing Comparative Examples 1 to 3 or the second complex containing Examples 1 and 3 to 4 was collected by centrifugation at a rotation speed of 10000 rpm for 3 minutes, and the second complex containing Example 2 was collected with a magnet and stayed on the magnet for at least 1 minute. The reaction solution was removed and the second complex was repetitively washed with 200 µL of a PBS solution two times.

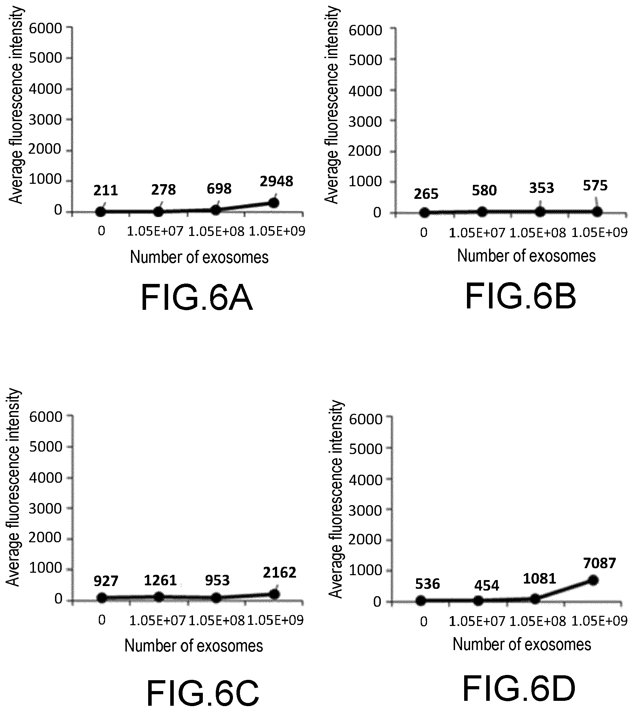

In this embodiment, the step in which fluorescence signal analysis was performed on the second complex to detect the expression of exosome surface antigens is generally as follows: 100 µL of a PBS solution is added to the washed second complex (i.e., the second complex containing Comparative Example 1, the second complex containing Example 1, or the second complex containing Example 2), and fluorescence signal analysis was performed with a flow cytometry analyzer to detect the expression (i.e., average fluorescence intensity) of exosome surface antigens. The results are as shown in FIG. 6A to FIG. 6L, FIG. 7A to FIG. 7C, and FIG. 8A to FIG. 8C.

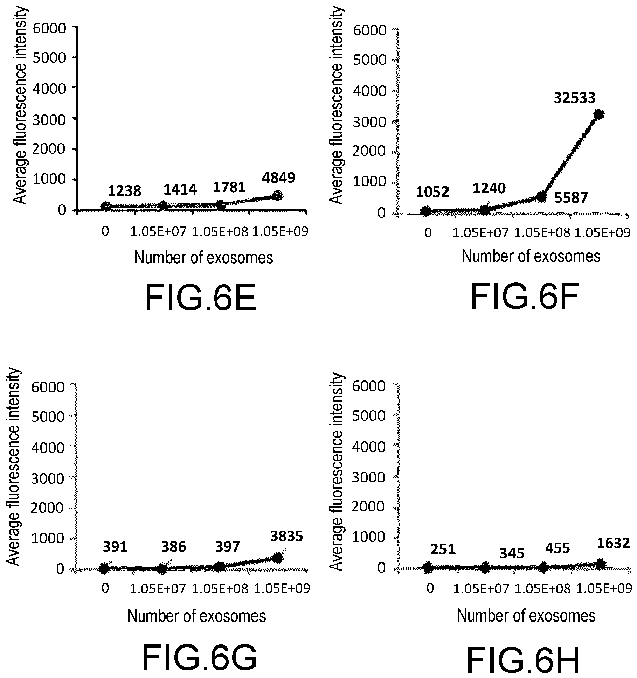

FIG. 6A to FIG. 6F are respectively graphs of CD9 expressions of exosomes of SKBr3 detected by using Comparative Examples 1 to 3 and Examples 1 and 3 to 4 coupled with anti-human CD63. Specifically, FIG. 6A, FIG. 6C and FIG. 6E are the results of detection with spherical microparticles having diameters of about 2.5 µm, 4.5 µm and 8.5 µm (Comparative Example 2, Comparative Example 3 and Comparative Example 1), respectively. FIG. 6B, FIG. 6D and FIG. 6F are the results of detection with knobby particles having diameters of about 2.5 µm, 4.5 µm, and 8.5 µm (Example 3, Example 4, and Example 1), respectively. According to the results of FIG. 6A and FIG. 6B, compared with the spherical microparticles with a diameter of 2.5 µm (Comparative Example 2), the knobby particles with a diameter of 2.5 µm (Example 3) may have a lower detected fluorescence signal due to uneven surface morphology. According to the results of FIG. 6C (or FIG. 6E), as the number of exosomes (0, 1.05×10⁷, 1.05×10⁸, or 1.05×10⁹) in Comparative Example 3 (or Comparative Example 1) increases, the number of exosomes that Comparative Example 3 (or Comparative Example 1) binds to increases, and the detected CD9 and average fluorescence intensity also increase. According to the results of FIG. 6D (or FIG. 6F), as the number of exosomes (0, 1.05×10⁷, 1.05×10⁸, or 1.05×10⁹) in Example 4 (or Example 1) increases, the number of exosomes that Example 4 (or Example 1) binds to increases, and the detected CD9 and average fluorescence intensity also increase. However, according to the results of FIG. 6C to FIG. 6F, compared to Comparative Example 3 (or Comparative Example 1) containing the spherical microparticle, since Example 4 (or Example 1) containing the knobby particle binds to more exosomes, more CD9 and greater average fluorescence intensity can be detected.

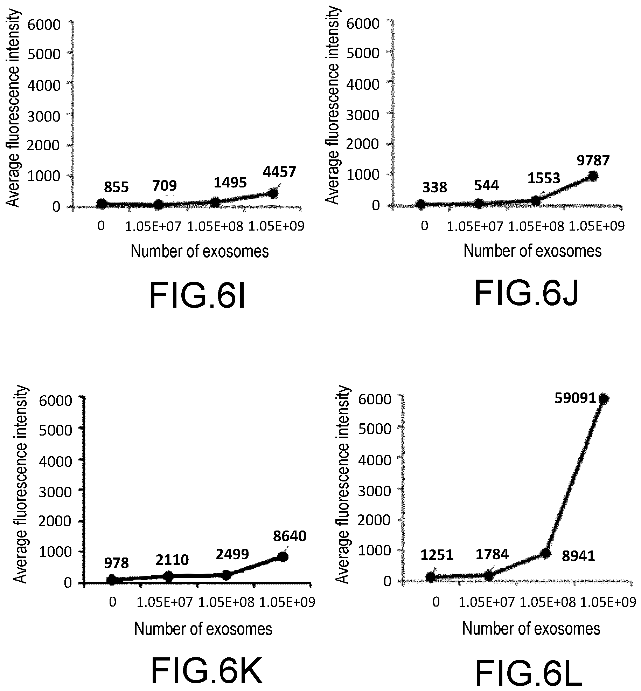

FIG. 6G to FIG. 6L are respectively graphs of CD9 expressions of exosomes of SKBr3 detected by using Comparative Examples 1 to 3 and Examples 1 and 3 to 4 coupled with anti-human CD9. Specifically, FIG. 6G, FIG. 6I and FIG. 6K are the results of detection with spherical microparticles having diameters of about 2.5 µm, 4.5 µm and 8.5 µm (Comparative Example 2, Comparative Example 3 and Comparative Example 1), respectively. FIG. 6H, FIG. 6J and FIG. 6L are the results of detection with knobby particles having diameters of about 2.5 µm, 4.5 µm, and 8.5 µm (Example 3, Example 4, and Example 1), respectively. According to the results of FIG. 6G and FIG. 6H, compared with the spherical microparticles with a diameter of 2.5 µm (Comparative Example 2), the knobby particles with a diameter of 2.5 µm (Example 3) may have a lower detected fluorescence signal due to uneven surface morphology. According to the results of FIG. 6I (or FIG. 6K), as the number of exosomes (0, 1.05×10⁷, 1.05×10⁸, or 1.05×10⁹) in Comparative Example 3 (or Comparative Example 1) increases, the number of exosomes that Comparative Example 3 (or Comparative Example 1) binds to increases, and the detected CD9 and average fluorescence intensity also increase. According to the results of FIG. 6J (or FIG. 6L), as the number of exosomes (0, 1.05×10⁷, 1.05×10⁸, or 1.05×10⁹) in Example 4 (or Example 1) increases, the number of exosomes that Example 4 (or Example 1) binds to increases, and the detected CD9 and average fluorescence intensity also increase. However, according to the results of FIG. 6I to FIG. 6L, compared to Comparative Example 3 (or Comparative Example 1) containing the spherical microparticle, since Example 4 (or Example 1) containing the knobby particle binds to more exosomes, more CD9 and greater average fluorescence intensity can be detected.

It should be noted that according to FIG. 6A to FIG. 6L, the larger the particle, the stronger the signal, as well as the larger the particle size and the more protruding shape have the best analysis signal. Using flow cytometry analyzer to detect microbeads with larger particle size and uniform size for analysis has better analysis signal.

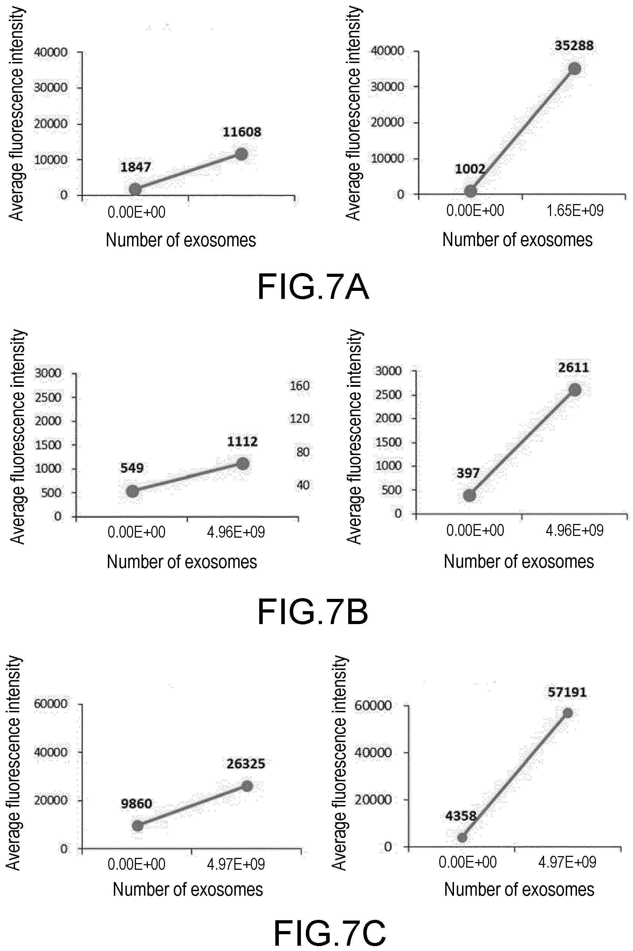

FIG. 7A to FIG. 7C are respectively graphs of CD9 expressions of exosomes of SKBr3 in different solutions detected by using Example 1 and Example 2 coupled with anti-human CD9. Specifically, exosomes of SKBr3 were first placed in a DMEM culture medium (i.e., supernatant collected from SKBr3 culture medium), a PBS solution (i.e., exosomes of SKBr3 purified and then placed in a PBS solution), or EDTA-containing blood plasma (i.e., exosomes of SKBr3 purified and then placed in EDTA-containing blood plasma). Next, the CD9 expression of the exosomes of SKBr3 in the DMEM culture medium was detected by using Example 1 and Example 2 coupled with anti-human CD9, and the results are shown in FIG. 7A. The CD9 expression of the exosomes of SKBr3 in the PBS solution was detected by using Example 1 and Example 2 coupled with anti-human CD9, and the results are shown in FIG. 7B. The CD9 expression of the exosomes of SKBr3 in the EDTA-containing blood plasma was detected by using Example 1 and Example 2 coupled with anti-human CD9, and the results are shown in FIG. 7C. The above-mentioned blood plasma was from healthy people.

According to the results of FIG. 7A, compared to Example 1 containing the knobby particle, since Example 2 containing the knobby magnetic particle binds to more exosomes located in the DMEM culture medium, more CD9 and greater average fluorescence intensity can be detected. According to the results of FIG. 7B, compared to Example 1 containing the knobby particle, since Example 2 containing the knobby magnetic particle binds to more exosomes located in the PBS solution, more CD9 and greater average fluorescence intensity can be detected. According to the results of FIG. 7C, compared to Example 1 containing the knobby particle, since Example 2 containing the knobby magnetic particle binds to more exosomes in the EDTA-containing blood plasma, more CD9 and greater average fluorescence intensity can be detected. Besides, according to the results of FIG. 7A to FIG. 7C, Example 1 containing the knobby particle or Example 2 containing the knobby magnetic particle may not only detect exosomes located in a PBS solution but also detect exosomes located in relatively complicated environments such as a DMEM culture medium or EDTA-containing blood plasma.

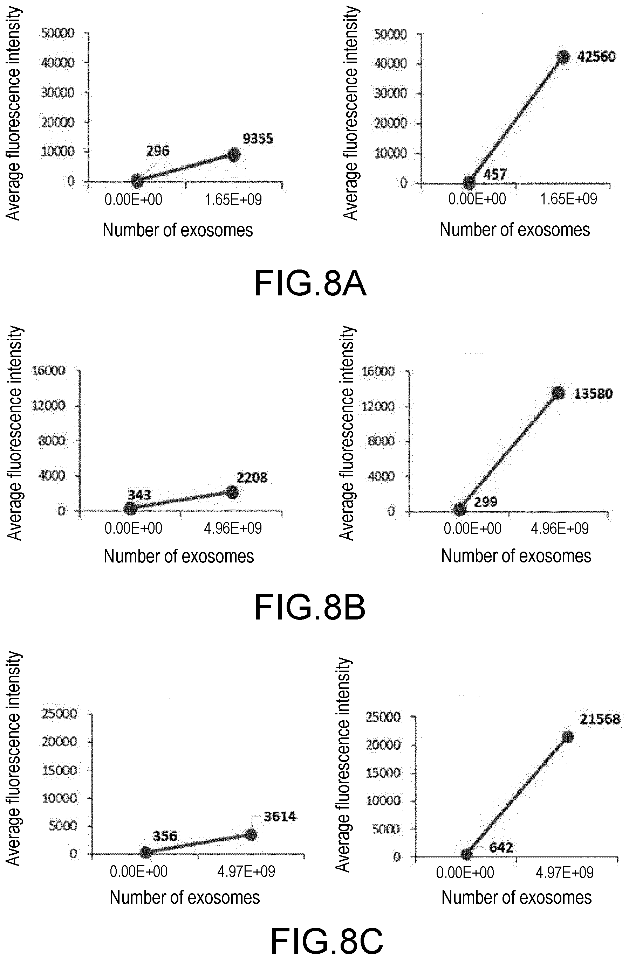

FIG. 8A to FIG. 8C are respectively graphs of CD81 expressions of exosomes of SKBr3 in different solutions detected by using Example 1 and Example 2 coupled with anti-human CD9. Specifically, exosomes of SKBr3 were first placed in a DMEM culture medium (supernatant collected from SKBr3 culture medium), a PBS solution (exosomes of SKBr3 purified and then placed in a PBS solution), or EDTA-containing blood plasma (exosomes of SKBr3 purified and then placed in EDTA-containing blood plasma). Next, the CD81 expression of the exosomes of SKBr3 in the DMEM culture medium was detected by using Example 1 and Example 2 coupled with anti-human CD9, and the results are shown in FIG. 8A. The CD81 expression of the exosomes of SKBr3 in the PBS solution was detected by using Example 1 and Example 2 coupled with anti-human CD9, and the results are shown in FIG. 8B. The CD81 expression of the exosomes of SKBr3 in the EDTA-containing blood plasma was detected by using Example 1 and Example 2 coupled with anti-human CD9, and the results are shown in FIG. 8C. The above-mentioned blood plasma was from healthy people.

According to the results of FIG. 8A, compared to Example 1 containing the knobby particle, since Example 2 containing the knobby magnetic particle binds with more exosomes in the DMEM culture medium, more CD81 and greater average fluorescence intensity can be detected. According to the result of FIG. 8B, compared to Example 1 containing the knobby particle, since Example 2 containing the knobby magnetic particle binds to more exosomes in the PBS solution, more CD81 and greater average fluorescence intensity can be detected. According to the results of FIG. 8C, compared to Example 1 containing the knobby particle, since Example 2 containing the knobby magnetic particle binds to more exosomes in the EDTA-containing blood plasma, more CD81 and greater average fluorescence intensity can be detected. Besides, according to the results of FIG. 8A to FIG. 8C, Example 1 containing the knobby particle or Example 2 containing the knobby magnetic particle may not only detect exosomes located in a PBS solution but also detect exosomes located in relatively complicated environments such as a DMEM culture medium or EDTA-containing blood plasma.

### Embodiment 4: detection of the exosome surface antigen on breast cancer cell lines SKBr3

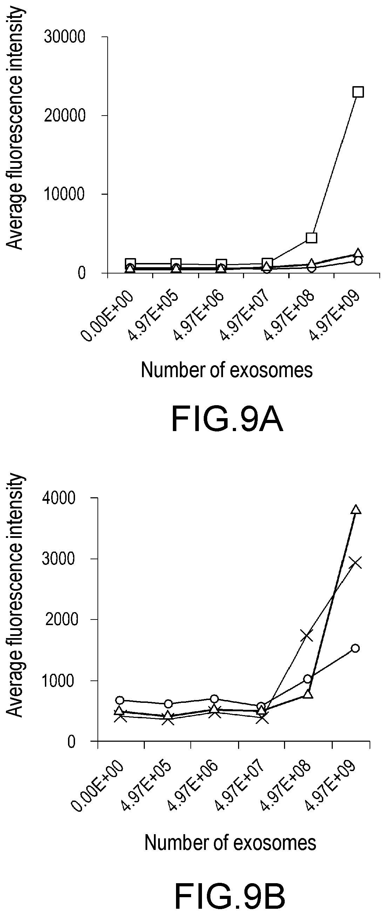

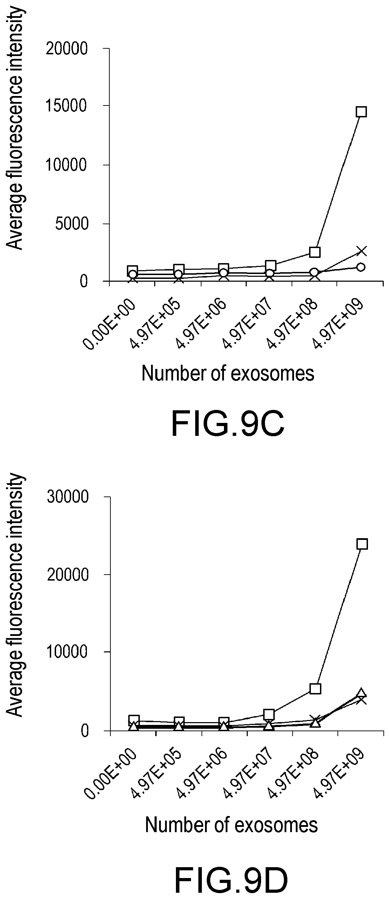

FIG. 9A to FIG. 9D are graphs of any three of CD9, CD29, CD63, and CD81 expressions of exosomes of SKBr3 detected at the same time by using Example 2 coupled with anti-human CD9. Specifically, in this embodiment, a plurality of types of surface antigens on exosomes of breast cancer cell lines SKBr3 were detected at the same time using an experimental procedure similar to that of Embodiment 3, which will thus not be repeatedly described herein. The difference between this embodiment and Embodiment 3 lies in that, in this embodiment, exosomes of SKBr3 were first placed in EDTA-containing blood plasma. Then, the CD9, CD63, and CD81 expressions of the exosomes were detected at the same time by using Example 2 coupled with anti-human CD9 and a plurality of types of second specific antibodies (anti-human CD9-Alexa488, anti-human CD63-PerCPCy5.5, and anti-human CD81-APC) carrying a plurality of types of fluorescent labels, and the results are shown in FIG. 9A. The CD29, CD63, and CD81 expressions of the exosomes were detected at the same time by using Example 2 coupled with anti-human CD9 and a plurality of types of second specific antibodies (anti-human CD29-PE, anti-human CD63-PerCPCy5.5, and anti-human CD81-APC) carrying a plurality of types of fluorescent labels, and the results are shown in FIG. 9B. The CD9, CD29, and CD63 expressions of the exosomes were detected at the same time by using Example 2 coupled with anti-human CD9 and a plurality of types of second specific antibodies (anti-human CD9-Alexa488, anti-human CD29-PE, and anti-human CD63-PerCPCy5.5) carrying a plurality of types of fluorescent labels, and the results are shown in FIG. 9C. The CD9, CD29, and CD81 expressions of the exosomes were detected at the same time by using Example 2 coupled with anti-human CD9 and a plurality of types of second specific antibodies (anti-human CD9-Alexa488, anti-human CD29-PE, and anti-human CD81-APC) carrying a plurality of types of fluorescent labels, and the results are shown in FIG. 9D. The above-mentioned blood plasma was from healthy people. Besides, in FIG. 9A to FIG. 9D, the CD9 expression of the exosomes is represented by the symbol □, the CD29 expression of the exosomes is represented by the symbol ×, the CD63 expression of the exosomes is represented by the symbol ○, and the CD81 expression of the exosomes is represented by the symbol △.

According to the results of FIG. 9A to FIG. 9D, even if the exosomes are located in relatively complicated environments such as EDTA-containing blood plasma, by the detection method of this embodiment, the expressions of any three of the exosome surface antigens (i.e., any three of CD9, CD29, CD63, and CD81) on the exosomes may still be detected at the same time by using Example 2 (the knobby magnetic particle coupled with anti-human CD9) and any three second specific antibodies (i.e., any three of anti-human CD9-Alexa488, anti-human CD29-PE, anti-human CD63-PerCPCy5.5, and anti-human CD81-APC) carrying fluorescent labels to detect multiple analytes at the same time. Besides, although detection for three kinds of analytes at the same time is taken as an example in this embodiment, the number of analytes that may be detected at the same time is not limited by the disclosure. That is, in some embodiments, two or more than three kinds of analytes may also be detected at the same time.

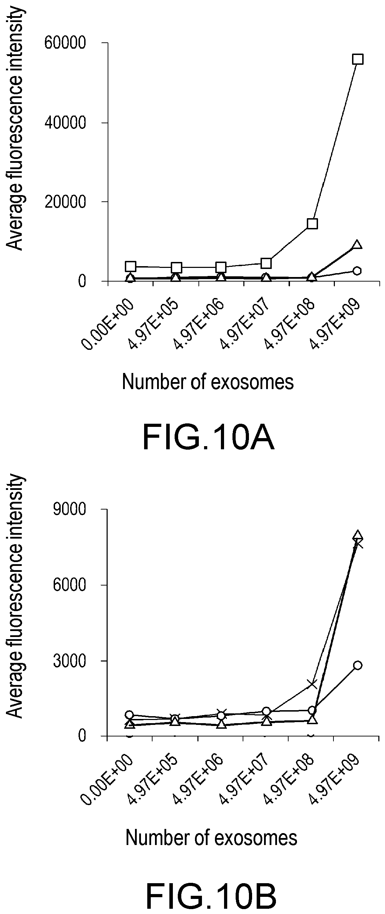

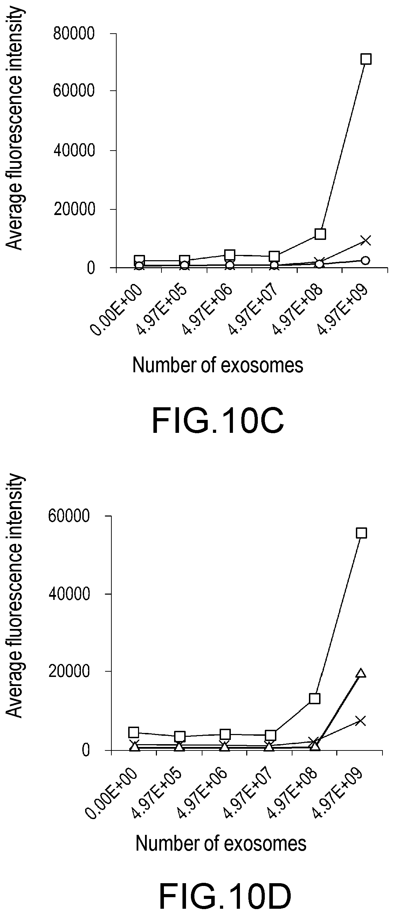

FIG. 10A to FIG. 10D are graphs of any three of CD9, CD29, CD63, and CD81 expressions of exosomes of SKBr3 detected at the same time by using Example 2 coupled with anti-human HER2. Specifically, exosomes of SKBr3 were first placed in EDTA-containing blood plasma. Then, the CD9, CD63, and CD81 expressions of the exosomes were detected at the same time by using Example 2 coupled with anti-human HER2 and a plurality of types of second specific antibodies (anti-human CD9-Alexa488, anti-human CD63-PerCPCy5.5, and anti-human CD81-APC) carrying a plurality of types of fluorescent labels, and the results are shown in FIG. 10A. The CD29, CD63, and CD81 expressions of the exosomes were detected at the same time by using Example 2 coupled with anti-human HER2 and a plurality of types of second specific antibodies (anti-human CD29-PE, anti-human CD63-PerCPCy5.5, and anti-human CD81-APC) carrying a plurality of types of fluorescent labels, and the results are shown in FIG. 10B. The CD9, CD29, and CD63 expressions of the exosomes were detected at the same time by using Example 2 coupled with anti-human HER2 and a plurality of types of second specific antibodies (anti-human CD9-Alexa488, anti-human CD29-PE, and anti-human CD63-PerCPCy5.5) carrying a plurality of types of fluorescent labels, and the results are shown in FIG. 10C. The CD9, CD29, and CD81 expressions of the exosomes were detected at the same time by using Example 2 coupled with anti-human HER2 and a plurality of types of second specific antibodies (anti-human CD9-Alexa488, anti-human CD29-PE, and anti-human CD81-APC) carrying a plurality of types of fluorescent labels, and the results are shown in FIG. 10D. The above-mentioned blood plasma was from healthy people. Besides, in FIG. 10A to FIG. 10D, the CD9 expression of the exosomes is represented by the symbol □, the CD29 expression of the exosomes is represented by the symbol ×, the CD63 expression of the exosomes is represented by the symbol ○, and the CD81 expression of the exosomes is represented by the symbol △.

According to the results of FIG. 10A to FIG. 10D, even if the exosomes are located in relatively complicated environments such as EDTA-containing blood plasma, by the detection method of this embodiment, the expressions of any three of the exosome surface antigens (i.e., any three of CD9, CD29, CD63, and CD81) on the exosomes may still be detected at the same time by using Example 2 (the knobby magnetic particle coupled with anti-human HER2) and any three second specific antibodies (i.e., any three of anti-human CD9-Alexa488, anti-human CD29-PE, anti-human CD63-PerCPCy5.5, and anti-human CD81-APC) carrying fluorescent labels to detect multiple analytes at the same time.

Besides, according to the comparisons of the results of FIG. 9A to FIG. 10A, of FIG. 9B to FIG. 10B, of FIG. 9C to FIG. 10C, and of FIG. 9D to FIG. 10D, when the expressions of the exosome surface antigens (i.e., CD9, CD29, CD63, and CD81) of SKBr3 are analyzed by using the knobby magnetic particle coupled with whichever of anti-human CD9 and anti-human HER2, high consistency exists between the detection results, indicating that the detection method with high accuracy and reduced errors is used in this embodiment.

In addition, according to the combined results of FIG. 9A to FIG. 9D and FIG. 10A to FIG. 10D, when the expressions of the exosome surface antigens (i.e., CD9, CD29, CD63, and CD81) is analyzed by using the knobby magnetic particle coupled with whichever of anti-human CD9 and anti-human HER2 binding to the exosomes of SKBr3 accompanied with any three kinds of second specific antibodies (i.e., any three of anti-human CD9-Alexa488, anti-human CD29-PE, anti-human CD63-PerCPCy5.5, and anti-human CD81-APC) carrying fluorescent labels, the CD9 expression is generally the greatest, and the CD81 expression is slightly greater than or similar to the CD63 expression and the CD29expression, indicating that even if there exist more than two variant parameters in the detection method of this embodiment, the obtained detection results still exhibit comparability.

### Embodiment 5: detection of exosome surface antigens on clinical specimens

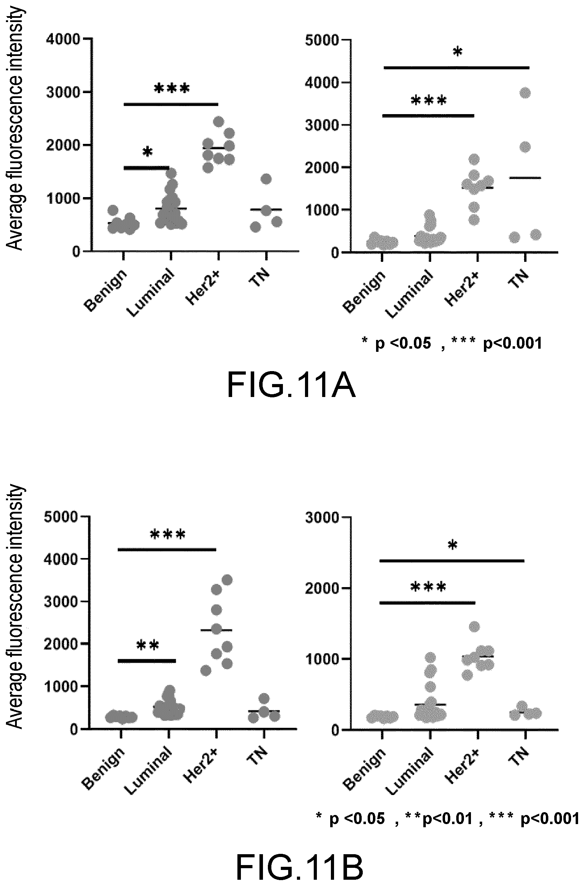

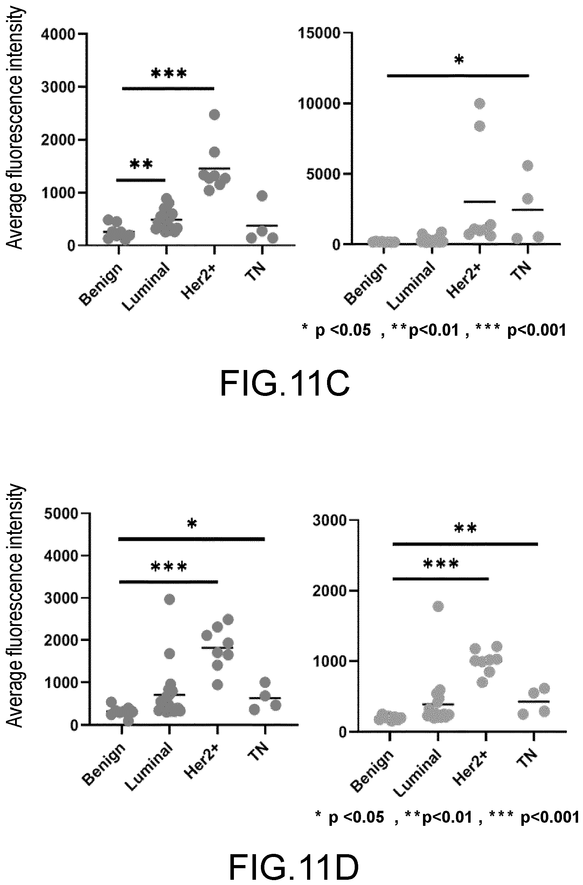

FIG. 11A to FIG. 11D are respectively graphs of CD9 and CD63 expressions of exosomes in blood plasma as a clinical specimen detected at the same time by using Example 2 coupled with different first specific antibodies. The first specific antibody is anti-human CD9, anti-human HER2, anti-human EGFR, or anti-human EpCAM. The clinical specimens was from blood plasma of 8 benign tumors, blood plasma of 20 luminal breast cancers, blood plasma of 8 HER2-positive type breast cancers (HER2), and blood plasma of 4 triple-negative (TN) breast cancers. The second specific antibodies carrying fluorescent labels are anti-human CD9-Alexa488 and anti-human CD63-PE. Specifically, in this embodiment, CD9 and CD63 of the exosome in the clinical specimens were detected at the same time using an experimental procedure similar to that of Embodiment 3, which will thus not be repeatedly described herein. The difference between this embodiment and Embodiment 3 lies in that, in this embodiment, exosomes in different clinical specimens were first placed in EDTA-containing blood plasma. Next, the CD9 and CD63 expressions of the exosomes were detected at the same time by using Example 2 coupled with anti-human CD9, and the results are shown in FIG. 11A. The CD9 and CD63 expressions of the exosomes were detected at the same time by using Example 2 coupled with anti-human HER2, and the results are shown in FIG. 11B. The CD9 and CD63 expressions of the exosomes were detected at the same time by using Example 2 coupled with anti-human EGFR, and the results are shown in FIG. 11C. The CD9 and CD63 expressions of the exosomes were detected at the same time by using Example 2 coupled with anti-human EpCAM, and the results are shown in FIG. 11D.

According to the results of FIG. 11A to FIG. 11D, in the detection method of this embodiment, for different clinical specimens, the expressions of two exosome surface antigens (i.e., CD9 and CD63) on the exosomes may still be detected at the same time by using Example 2 (i.e., the knobby magnetic particle coupled with anti-human CD9, anti-human HER2, anti-human EGFR, or anti-human EpCAM) and two second specific antibodies (i.e., anti-human CD9-Alexa488 and anti-human CD63-PE) carrying fluorescent labels to detect multiple analytes at the same time.

### Embodiment 6: nucleic acid detection

### Method of probe coupling

In this embodiment, a probe (avian influenza virus (AIV) probe) was first coupled to the knobby magnetic particle of this disclosure. Next, a biotin-labeled nucleic acid sequence (AB nucleic acid to be analyzed or AIV nucleic acid to be analyzed) was added for analysis.

In this embodiment, the AIV probe has a nucleic acid sequence of sequence identification number: 1, the AB nucleic acid to be analyzed has a nucleic acid sequence of sequence identification number: 2, and the AIV nucleic acid to be analyzed has a nucleic acid sequence of sequence identification number: 3. The sequences are shown in detail in the table below.

| | |
|---|---|
| AIV probe (SEQ ID NO: 1) | 5'-gtctacgctgcagtcctcgctcactgggca |
| AB nucleic acid to be analyzed (SEQ ID NO: 2) | 5'-aaaaaaaaaaaaaatcctggagctaagtccgta |
| AIV nucleic acid to be analyzed (SEQ ID NO: 3) | 5'-caagaccaatcctgtcacctctgactaaggggattttagggtttgtgttcacgctc accgtgcccagtgagcgaggactgcagcgtagac |

In this embodiment, the step in which the AIV probe was coupled to the knobby magnetic particle is as follows: (1) 1×10⁶ counts of knobby magnetic particles surface-modified with amine groups were washed with 200 µL of a MES buffer solution three times. Next, 20 mg of EDC, 20 mg of NHS, and 4 mg of PAA were dissolved in 400 µL of a MES buffer solution and mixed with the washed knobby magnetic particles. Then, at room temperature, the mixing was performed with a vortex mixer at a rotation speed of 1000 rpm for reaction for 30 minutes. Next, the knobby magnetic particles were collected with a magnet and stayed on the magnet for at least 1 minute. The reaction solution was removed and the knobby magnetic particles were repetitively washed with 200 µL of a MES buffer solution three times, then added with 20 µL of AIV probes into 80 µL of a PBS solution, and then reacted at room temperature at a rotation speed of 1000 rpm for 2 hours. (2) After the reaction was completed, the knobby magnetic particles were collected with a magnet and stayed on the magnet for at least 1 minute. The reaction solution was removed, the knobby magnetic particles were washed with a Tris solution (25 mM of Tris, pH 7.4) three times for at least 1 minute each time. Lastly, the knobby magnetic particles coupled with the AIV probe are dispersed in 100µL of a Tris solution. (3) The concentration of the knobby magnetic particles was calculated by using an automated cell counter, and knobby magnetic particles coupled with AIV probes on the surface with a concentration of about 3 to 8×10⁶/mL were obtained.

### Nucleic acid detection performed with knobby magnetic particles coupled with probes

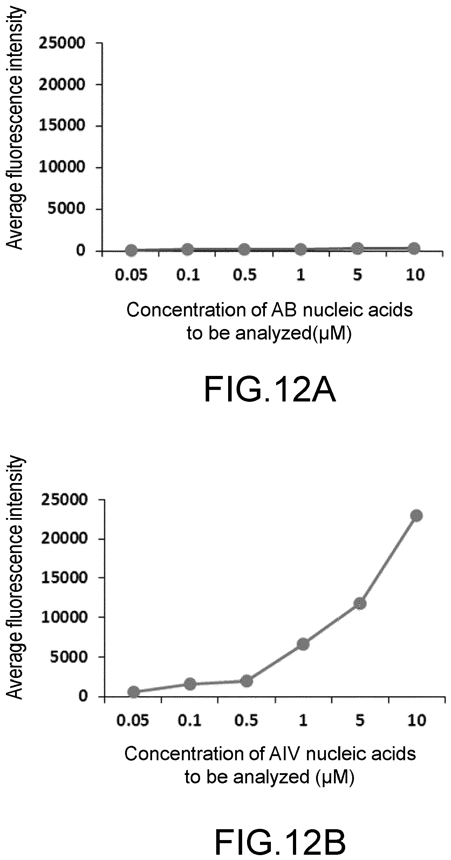

In this embodiment, the step in which the AB nucleic acid to be analyzed or the AIV nucleic acid to be analyzed, carrying biotin, was detected by using the knobby magnetic particles coupled with the AIV probe is generally as follows: (1) 30 µL of a hybridization solution (TEGO hybridization solution) was mixed with 30 µL of a solution of AB nucleic acids to be analyzed or AIV nucleic acids to be analyzed, carrying biotin, with different concentrations (0.05 µM, 0.1 µM, 0.5 µM, 5 µM, 10 µM). Next, 50 µL (1250 counts) of the knobby magnetic particles coupled with the AIV probe on the surface were added, and mixing was performed at 60°C with a vortex mixer at a rotation speed of 1000 rpm for reaction for 30 minutes. Matching and binding with specificity were performed between the AIV nucleic acid to be analyzed carrying biotin and the AIV probe in the knobby magnetic particles coupled with the AIV probe to form the first complex. After the reaction was completed, the knobby magnetic particles were collected with a magnet, and then, after the reaction solution was removed, were washed with 200 µL of a PBS solution two times. (2) 100 µL of an anti-biotin antibody (anti-biotin PE) carrying fluorescent labels were added to the knobby magnetic particles, and mixing was performed for reaction at room temperature for 30 minutes. The anti-biotin antibody carrying fluorescent labels bound to the biotin in the first complex to form the second complex. After the reaction was completed, the knobby magnetic particles were collected with a magnet, and, after the reaction solution was removed, were repetitively washed with 200 µL of a PBS buffer solution (PBS solution, pH 7.4) two times. (3) After washing, 150 µL of a PBS buffer solution was added and fluorescence signal analysis was performed with a flow cytometry analyzer. The results are shown in FIG. 12A and FIG. 12B.

As shown in FIG. 12A, since the AB nucleic acid to be analyzed carrying biotin does not bind to the AIV probe in the knobby magnetic particles coupled with the AIV probe, the first complex is thus not formed, and the second complex also is thus not formed. As a result, the measured average fluorescence intensity approaches zero.

As shown in FIG. 12B, matching and binding with specificity were performed between the AIV nucleic acid to be analyzed carrying biotin and the AIV probe in the knobby magnetic particles coupled with the AIV probe, the first complex was formed, and the biotin in the first complex bound to the anti-biotin antibody carrying fluorescent labels to form the second complex. Accordingly, the average fluorescence intensity can be measured. Besides, the value of average fluorescence intensity basically increases as the concentration of AIV nucleic acid to be analyzed carrying biotin increases.

In summary of the foregoing, in the microparticle of the disclosure, by disposing the first protrusions having irregular shapes on the surface of the body, the surface area of the microparticle (i.e., the sum of the surface area of the body and the surface area of the first protrusions) is increased. Accordingly, the microparticle of the disclosure can be coupled with more first ligands to recognize and bind to more targets, and the strength of detection signal can be increased and the detection sensitivity can be improved.

Besides, in the disclosure, compared to the knobby particle, since the surface of the magnetic substance layer of the knobby magnetic particle is a rough surface (or has small protrusions), the surface (i.e., the surface of the body and the surface of the first protrusions) of the knobby magnetic particle is also a rough surface. As a result, the knobby magnetic particle has a greater surface area. Further, the knobby magnetic particle can be coupled with more first ligands, and recognize and bind to more targets to increase the strength of detection signal and improve the detection sensitivity. Moreover, since the knobby magnetic particle is magnetic, the detection time can be reduced, and the efficiency and convenience of detection can be improved.

## Claims

1. A detection method of multiple analytes (130a, 130b, 130c), comprising:
providing a knobby microparticle (110), wherein the knobby microparticle (110) is coupled with at least one type of first ligand (120), and the knobby microparticle (110) comprises:
a body (111) comprising a copolymer core (113), a polymer layer (114), and a silicon-based layer (115) from the inside to the outside;
a plurality of first protrusions (112) formed on a surface (111a) of the body (111); and
a plurality of second protrusions (113b) are formed on a surface (113a) of the copolymer core (113);
mixing the knobby microparticle (110) with a specimen (S) comprising a plurality of types of analytes (130a, 130b, 130c) to form a first complex (140);
mixing the first complex (140) with a plurality of types of second ligands (150a, 150b, 150c) carrying a plurality of types of first labels (151a, 151b, 151c), such that the plurality of types of second ligands (150a, 150b, 150c) bind to the plurality of types of analytes (130a, 130b, 130c) in the first complex (140) and form a second complex (160); and
detecting the plurality of types of first labels (151a, 151b, 151c) in the second complex (160), wherein a ratio of an average volume (V1) of the first protrusions (112) to an average volume (V2) of the body (111) is 1×10⁻⁷ to 2×10⁻², and a total volume of the first protrusions (112) to an overall volume of the knobby microparticle (110) is 1×10⁻¹ to 6×10⁻¹.

2. The method according to claim 1, wherein an average height (H2) of the second protrusions (113b) is 100 nanometers to 5000 nanometers.

3. The method according to claim 1, wherein the knobby microparticle (110) further comprises a magnetic substance layer (116) between the polymer layer (114) and the silicon-based layer (115).

4. The method according to claim 1, wherein a ratio of an average height (H1) of the first protrusions (112) to an average diameter (D1) of the body (111) is 0.005 to 0.25.

5. The method according to claim 1, wherein an average number of the first protrusions (112) is 5 to 500, and an average diameter (D) of the knobby microparticle (110) is 1 µm to 20 µm.

6. The method according to claim 1, wherein the knobby microparticle (110) is non-spherical.

7. The method according to claim 1, wherein the plurality of types of analytes (130a, 130b, 130c) are located on a surface (S1) of the specimen (S), and the step of forming the first complex comprises (140) performing the at least one first ligand (120) to recognize and directly bind to a target (T) located on the surface (Sl) of the specimen (S).

8. The method according to claim 7, wherein the at least one first ligand (120) comprises a first specific antibody, and the first specific antibody comprises an antibody against a surface antigen on the human exosome, an antibody against a surface antigen on the human blood cell, an antibody against a surface antigen on the human immune cell, an antibody against a surface antigen on the human tumor cell, or a combination thereof.

9. The method according to claim 7, wherein the specimen (S) comprises a human exosome, a human blood cell, a human immune cell, a human tumor cell, or a combination thereof, and the plurality of types of analytes comprise a surface antigen on the human exosome, a surface antigen on the human blood cell, a surface antigen on the human immune cell, a surface antigen on the human tumor cell, or a combination thereof.

10. The method according to claim 7, wherein the plurality of types of second ligands (150a, 150b, 150c) comprise a plurality of types of second specific antibodies, and the plurality of types of second specific antibodies comprise an antibody against a surface antigen on the human exosome, an antibody against a surface antigen on the human blood cell, an antibody against a surface antigen on the human immune cell, an antibody against a surface antigen on the human tumor cell, or a combination thereof.

11. The method according to claim 1, wherein the at least one first ligand (120) comprises a plurality of types of first ligands (120a, 120b, 120c), and the step forming the first complex (140) comprises performing the plurality of types of first ligands (120a, 120b, 120c) to recognize and directly bind to the plurality of types of analytes (130a, 130b, 130c).

12. The method according to claim 11, wherein the plurality of types of first ligands (120a, 120b, 120c) comprise a plurality of types of nucleic acid probes, and the plurality of types of nucleic acid probes comprise a plurality of types of primers or aptamers, the plurality of types of analytes (130a, 130b, 130c) comprise a plurality of types of nucleic acid sequences (131a, 131b, 131c) carrying a plurality of types of second labels (132a, 132b, 132c), and the plurality of types of second labels comprise biotin, a plurality of types of antigenic epitopes, or a combination thereof.

13. A detection method of multiple analytes (130a, 130b, 130c), comprising:
providing a knobby microparticle (110), wherein the knobby microparticle (110) is coupled with a plurality of types of ligands (120a, 120b, 120c), and the knobby microparticle (110) comprises:
a body (111) comprising a copolymer core (113), a polymer layer (114), and a silicon-based layer (115) from the inside to the outside; [[and]]
a plurality of protrusions (112) formed on a surface (111a) of the body (111); and
a plurality of second protrusions (113b) are formed on a surface (113a) of the copolymer core (113);
mixing the knobby microparticle (110) with a specimen (S) comprising a plurality of types of analytes (130a, 130b, 130c) to form a complex (140), wherein the plurality of types of analytes (130a, 130b, 130c) carry a plurality of types of labels (132a, 132b, 132c); and
detecting the plurality of types of labels (132a, 132b, 132c) in the complex (140), wherein a ratio of an average volume (V1) of the first protrusions (112) to an average volume (V2) of the body (111) is 1×10⁻⁷ to 2×10⁻², and a total volume of the first protrusions (112) to an overall volume of the knobby microparticle (110) is 1×10⁻¹ to 6×10⁻¹.

14. The method according to claim 13, wherein the plurality of types of ligands (120a, 120b, 120c) comprise a plurality of types of nucleic acid probes, the plurality of types of labels (132a, 132b, 132c) comprise a plurality of types of fluorescent labels, a plurality of types of luminescent labels, or a combination thereof, and the plurality of types of analytes (130a, 130b, 130c) comprise a plurality of types of nucleic acid sequences (131a, 131b, 131c).

## Patentansprüche

1. Erfassungsverfahren mehrerer Analyten (130a, 130b, 130c), umfassend:
Bereitstellen eines höckerigen Mikropartikels (110), wobei das höckerige Mikropartikel (110) mit mindestens einem Typ eines ersten Liganden (120) gekoppelt ist, und das höckerige Mikropartikel (110) umfasst:
einen Körper (111), der einen Copolymerkern (113), eine Polymerschicht (114) und eine siliziumbasierte Schicht (115), von innen nach außen, umfasst;
eine Vielzahl von ersten Vorsprüngen (112), die auf einer Oberfläche (111a) des Körpers (111) ausgebildet sind; und
eine Vielzahl von zweiten Vorsprüngen (113b), die auf einer Oberfläche (113a) des Copolymerkerns (113) ausgebildet sind;
Mischen des höckerigen Mikropartikels (110) mit einer Probe (S), umfassend eine Vielzahl von Typen von Analyten (130a, 130b, 130c), um einen ersten Komplex (140) zu bilden;
Mischen des ersten Komplexes (140) mit einer Vielzahl von Typen von zweiten Liganden (150a, 150b, 150c), die eine Vielzahl von Typen von ersten Markierungen (151a, 151b, 151c) tragen, so dass die Vielzahl von Typen von zweiten Liganden (150a, 150b, 150c) an die Vielzahl von Typen von Analyten (130a, 130b, 130c) in dem ersten Komplex (140) binden und einen zweiten Komplex (160) bilden; und
Erfassen der Vielzahl von Typen von ersten Markierungen (151a, 151b, 151c) in dem zweiten Komplex (160), wobei ein Verhältnis eines durchschnittlichen Volumens (V1) der ersten Vorsprünge (112) zu einem durchschnittlichen Volumen (V2) des Körpers (111) 1 × 10⁻⁷ bis 2 × 10⁻² und ein Gesamtvolumen der ersten Vorsprünge (112) zu einem Gesamtvolumen des höckerigen Mikropartikels (110) 1 × 10⁻¹ bis 6 × 10⁻¹ beträgt.

2. Verfahren gemäß Anspruch 1, wobei eine durchschnittliche Höhe (H2) der zweiten Vorsprünge (113b) 100 Nanometer bis 5000 Nanometer beträgt.

3. Verfahren gemäß Anspruch 1, wobei das höckerige Mikropartikel (110) ferner eine magnetische Substanzschicht (116) zwischen der Polymerschicht (114) und der siliziumbasierten Schicht (115) umfasst.

4. Verfahren gemäß Anspruch 1, wobei das Verhältnis einer durchschnittlichen Höhe (H1) der ersten Vorsprünge (112) zu einem durchschnittlichen Durchmesser (D1) des Körpers (111) 0,005 bis 0,25 beträgt.

5. Verfahren gemäß Anspruch 1, wobei die durchschnittliche Anzahl der ersten Vorsprünge (112) 5 bis 500 beträgt und der durchschnittliche Durchmesser (D) des höckerigen Mikropartikels (110) 1 µm bis 20 µm beträgt.

6. Verfahren gemäß Anspruch 1, wobei das höckerige Mikropartikel (110) nicht kugelförmig ist.

7. Verfahren gemäß Anspruch 1, wobei sich die Vielzahl von Typen von Analyten (130a, 130b, 130c) auf einer Oberfläche (S1) der Probe (S) befinden und der Schritt des Bildens des ersten Komplexes das Durchführen des mindestens einen ersten Liganden (120) zum Erkennen und direkten Binden an ein auf der Oberfläche (S1) der Probe (S) befindliches Ziel (T) umfasst (140).

8. Verfahren gemäß Anspruch 7, wobei der mindestens eine erste Ligand (120) einen ersten spezifischen Antikörper umfasst, und der erste spezifische Antikörper einen Antikörper gegen ein Oberflächenantigen auf dem menschlichen Exosom, einen Antikörper gegen ein Oberflächenantigen auf der menschlichen Blutzelle, einen Antikörper gegen ein Oberflächenantigen auf der menschlichen Immunzelle, einen Antikörper gegen ein Oberflächenantigen auf der menschlichen Tumorzelle oder eine Kombination davon umfasst.

9. Verfahren gemäß Anspruch 7, wobei die Probe (S) ein menschliches Exosom, eine menschliche Blutzelle, eine menschliche Immunzelle, eine menschliche Tumorzelle oder eine Kombination davon umfasst, und die Vielzahl von Typen von Analyten ein Oberflächenantigen auf dem menschlichen Exosom, ein Oberflächenantigen auf der menschlichen Blutzelle, ein Oberflächenantigen auf der menschlichen Immunzelle, ein Oberflächenantigen auf der menschlichen Tumorzelle oder eine Kombination davon umfassen.

10. Verfahren gemäß Anspruch 7, wobei die Vielzahl von Typen von zweiten Liganden (150a, 150b, 150c) eine Vielzahl von Typen von zweiten spezifischen Antikörpern umfassen, und die Vielzahl von Typen von zweiten spezifischen Antikörpern einen Antikörper gegen ein Oberflächenantigen auf dem menschlichen Exosom, einen Antikörper gegen ein Oberflächenantigen auf der menschlichen Blutzelle, einen Antikörper gegen ein Oberflächenantigen auf der menschlichen Immunzelle, einen Antikörper gegen ein Oberflächenantigen auf der menschlichen Tumorzelle oder eine Kombination davon umfassen.

11. Verfahren gemäß Anspruch 1, wobei der mindestens eine erste Ligand (120) eine Vielzahl von Typen von ersten Liganden (120a, 120b, 120c) umfasst, und der Schritt des Bildens des ersten Komplexes (140) das Durchführen der Vielzahl von Typen von ersten Liganden (120a, 120b, 120c) umfasst, um die Vielzahl von Typen von Analyten (130a, 130b, 130c) zu erkennen und direkt an sie zu binden.

12. Verfahren gemäß Anspruch 11, wobei die Vielzahl von Typen von ersten Liganden (120a, 120b, 120c) eine Vielzahl von Typen von Nukleinsäuresonden umfassen, und die Vielzahl von Typen von Nukleinsäuresonden eine Vielzahl von Typen von Primern oder Aptameren umfassen, die Vielzahl von Typen von Analyten (130a, 130b, 130c) eine Vielzahl von Typen von Nukleinsäuresequenzen (131a, 131b, 131c) umfassen, die eine Vielzahl von Typen von zweiten Markierungen (132a, 132b, 132c) tragen, und die Vielzahl von Typen von zweiten Markierungen Biotin, eine Vielzahl von Typen von antigenen Epitopen oder eine Kombination davon umfassen.

13. Erfassungsverfahren mehrerer Analyten (130a, 130b, 130c), umfassend:
Bereitstellen eines höckerigen Mikropartikels (110), wobei das höckerige Mikropartikel (110) mit einer Vielzahl von Typen von Liganden (120a, 120b, 120c) gekoppelt ist, und das höckerige Mikropartikel (110) umfasst:
einen Körper (111), der einen Copolymerkern (113), eine Polymerschicht (114) und eine siliziumbasierte Schicht (115) von innen nach außen umfasst; [[und]]
eine Vielzahl von Vorsprüngen (112), die auf einer Oberfläche (111a) des Körpers (111) ausgebildet sind; und
eine Vielzahl von zweiten Vorsprüngen (113b), die auf einer Oberfläche (113a) des Copolymerkerns (113) ausgebildet sind;
Mischen des höckerigen Mikropartikels (110) mit einer Probe (S), die eine Vielzahl von Typen von Analyten (130a, 130b, 130c) umfasst, um einen Komplex (140) zu bilden, wobei die Vielzahl von Typen von Analyten (130a, 130b, 130c) eine Vielzahl von Typen von Markierungen (132a, 132b, 132c) tragen; und
Erfassen der Vielzahl von Typen von Markierungen (132a, 132b, 132c) in dem Komplex (140), wobei ein Verhältnis eines durchschnittlichen Volumens (V1) der ersten Vorsprünge (112) zu einem durchschnittlichen Volumen (V2) des Körpers (111) 1 × 10⁻⁷ bis 2 × 10⁻² und ein Gesamtvolumen der ersten Vorsprünge (112) zu einem Gesamtvolumen des höckerigen Mikropartikels (110) 1 × 10⁻¹ bis 6 × 10⁻¹ beträgt.

14. Verfahren gemäß Anspruch 13, wobei die Vielzahl von Typen von Liganden (120a, 120b, 120c) eine Vielzahl von Typen von Nukleinsäuresonden umfassen, die Vielzahl von Typen von Markierungen (132a, 132b, 132c) eine Vielzahl von Typen von Fluoreszenzmarkierungen, eine Vielzahl von Typen von Lumineszenzmarkierungen oder eine Kombination davon umfassen, und die Vielzahl von Typen von Analyten (130a, 130b, 130c) eine Vielzahl von Typen von Nukleinsäuresequenzen (131a, 131b, 131c) umfassen.

## Revendications

1. Un procédé de détection de multiples analytes (130a, 130b, 130c), comprenant :
le fait de fournir une microparticule à excroissances (110), la microparticule à excroissances (110) étant reliée à au moins un type de premier ligand (120), et la microparticule à excroissances (110) comprenant :
un corps (111) comprenant, de l'intérieur vers l'extérieur, un noyau copolymère (113), une couche polymère (114) et une couche à base de silicium (115) ;
une pluralité de premières saillies (112) formées sur une surface (111a) du corps (111) ; et
une pluralité de deuxièmes saillies (113b) sont formées sur une surface (113a) du noyau copolymère (113) ;
le fait de mélanger la microparticule à excroissances (110) avec un échantillon (S) comprenant une pluralité de types d'analytes (130a, 130b, 130c) pour former un premier complexe (140) ;
le fait de mélanger le premier complexe (140) avec une pluralité de types de deuxièmes ligands (150a, 150b, 150c) portant une pluralité de types de premiers marqueurs (151a, 151b, 151c), de telle sorte que les ligands de la pluralité de types de deuxièmes ligands (150a, 150b, 150c) se lient à la pluralité de types d'analytes (130a, 130b, 130c) dans le premier complexe (140) et forment un deuxième complexe (160) ; et
le fait de détecter la pluralité de types de premières étiquettes (151a, 151b, 151c) dans le deuxième complexe (160), un rapport d'un volume moyen (V1) des premières saillies (112) à un volume moyen (V2) du corps (111) étant compris entre 1×10⁻⁷ et 2×10⁻², et le volume total des premières saillies (112) par rapport au volume global de la microparticule à excroissances (110) est compris entre 1×10⁻¹ et 6×10⁻¹.

2. Le procédé selon la revendication 1, dans lequel la hauteur moyenne (H2) des deuxièmes saillies (113b) va de 100 nanomètres à 5000 nanomètres.

3. Le procédé selon la revendication 1, dans lequel la microparticule à excroissances (110) comprend en outre une couche de substance magnétique (116) entre la couche polymère (114) et la couche à base de silicium (115).

4. Le procédé selon la revendication 1, dans lequel le rapport d'une hauteur moyenne (H1) des premières saillies (112) sur un diamètre moyen (D1) du corps (111) va de 0,005 à 0,25.

5. Le procédé selon la revendication 1, dans lequel le nombre moyen de premières saillies (112) est de 5 à 500, et le diamètre moyen (D) de la microparticule à excroissances (110) va de 1 µm à 20 µm.

6. Le procédé selon la revendication 1, dans lequel la microparticule à excroissances (110) est non sphérique.

7. Le procédé selon la revendication 1, dans lequel les types d'analytes de la pluralité de types d'analytes (130a, 130b, 130c) sont situés sur une surface (S1) de l'échantillon (S), et l'étape de formation du premier complexe comprend (140) le fait de mettre en oeuvre le ou les premiers ligands (120) pour reconnaître une cible (T) située sur la surface (S1) de l'échantillon (S) et se lier directement à celle-ci.

8. Le procédé selon la revendication 7, dans lequel ledit au moins un premier ligand (120) comprend un premier anticorps spécifique, et le premier anticorps spécifique comprend un anticorps contre un antigène de surface sur l'exosome humain, un anticorps contre un antigène de surface sur une cellule sanguine humaine, un anticorps contre un antigène de surface sur la cellule immunitaire humaine, un anticorps contre un antigène de surface sur la cellule tumorale humaine, ou une combinaison de ceux-ci.

9. Le procédé selon la revendication 7, dans lequel l'échantillon (S) comprend un exosome humain, une cellule sanguine humaine, une cellule immunitaire humaine, une cellule tumorale humaine, ou une combinaison de ceux-ci, et les analytes de la pluralité de types d'analytes comprennent un antigène de surface sur l'exosome humain, un antigène de surface sur la cellule sanguine humaine, un antigène de surface sur la cellule immunitaire humaine, un antigène de surface sur la cellule tumorale humaine, ou une combinaison de ceux-ci.

10. Le procédé selon la revendication 7, dans lequel les ligands de la pluralité de types de deuxièmes ligands (150a, 150b, 150c) comprennent une pluralité de types de deuxièmes anticorps spécifiques, et les ligands de la pluralité de types de deuxièmes ligands spécifiques comprennent un anticorps contre un antigène de surface sur l'exosome humain, un anticorps contre un antigène de surface sur la cellule sanguine humaine, un anticorps contre un antigène de surface sur la cellule immunitaire humaine, un anticorps contre un antigène de surface sur la cellule tumorale humaine, ou une combinaison de ceux-ci.

11. Le procédé selon la revendication 1, dans lequel ledit au moins un premier ligand (120) comprend une pluralité de types de premiers ligands (120a, 120b, 120c), et l'étape de formation du premier complexe (140) comprend la mise en oeuvre de la pluralité de types de premiers ligands (120a, 120b, 120c) pour reconnaître la pluralité de types d'analytes (130a, 130b, 130c) et se lier directement à ceux-ci.

12. Le procédé selon la revendication 11, dans lequel les ligands de la pluralité de types de premiers ligands (120a, 120b, 120c) comprennent une pluralité de types de sondes d'acide nucléique, et les sondes de la pluralité de types de sondes d'acide nucléique comprennent une pluralité de types d'amorces ou des aptamères, les analytes de la pluralité de types d'analytes (130a, 130b, 130c) comprennent une pluralité de types de séquences d'acide nucléique (131a, 131b, 131c) portant une pluralité de types de deuxièmes marqueurs (132a, 132b, 132c), et les types de la pluralité de types de deuxièmes marqueurs comprennent de la biotine, une pluralité de types d'épitopes antigéniques, ou une combinaison de ceux-ci.

13. Un procédé de détection de plusieurs analytes (130a, 130b, 130c), comprenant :
le fait de fournir une microparticule à excroissances (110), la microparticule à excroissances (110) étant reliée à une pluralité de types de ligands (120a, 120b, 120c), et la microparticule à excroissances (110) comprend :
un corps (111) comprenant, de l'intérieur vers l'extérieur, un noyau copolymère (113), une couche polymère (114) et une couche à base de silicium (115) ;
une pluralité de saillies (112) formées sur une surface (111a) du corps (111) ; et
les saillies d'une pluralité de deuxièmes saillies (113b) sont formées sur une surface (113a) du noyau copolymère (113) ;
le fait de mélanger la microparticule à excroissances (110) avec un échantillon (S) comprenant une pluralité de types d'analytes (130a, 130b, 130c) pour former un complexe (140), les types d'analytes de la pluralité de types d'analytes (130a, 130b, 130c) portant une pluralité de types d'étiquettes (132a, 132b, 132c) ; et
le fait de détecter la pluralité de types d'étiquettes (132a, 132b, 132c) dans le complexe (140), un rapport d'un volume moyen (V1) des premières saillies (112) à un volume moyen (V2) du corps (111) étant de 1×10⁻⁷ à 2×10⁻², et un volume total des premières saillies (1 à 12) par rapport à un volume global de la microparticule à excroissances (110) étant de 1×10⁻¹ à 6×10⁻¹.

14. Le procédé selon la revendication 13, dans lequel les types de ligands de la pluralité de types de ligands (120a, 120b, 120c) comprennent une pluralité de types de sondes d'acide nucléique, les types de marqueurs de la pluralité de types de marqueurs (132a, 132b, 132c) comprennent une pluralité de types de marqueurs fluorescents, une pluralité de types de marqueurs luminescents, ou une combinaison de ceux-ci, et les types d'analytes de la pluralité de types d'analytes (130a, 130b, 130c) comprennent une pluralité de types de séquences d'acide nucléique (131a, 131b, 131c).
